# EUROPEAN PATENT SPECIFICATION

(11) **EP 1 648 502 B1**
(45) Date of publication and mention of the grant of the patent: **01.12.2010**
(21) Application number: 04763141.1
(22) Date of filing: 09.07.2004
(51) Int. Cl.: A61K 39/29, C07K 14/18

(54) **HCV VACCINES**
HCV-VAKZIN
VACCINS CONTRE LE VIRUS DE L'HEPATITE C

(30) Priority: 11.07.2003 EP 03450171; 12.03.2004 EP 04450062
(43) Date of publication of application: 26.04.2006
(60) Divisional application: 10179922.9
(73) Proprietor: Intercell AG, 1030 Vienna (AT)
(72) Inventor: BUSCHLE, Michael, Edinburgh EH4 6LS (GB); FRISCH, Jürgen, 35041 Marburg (DE); KLADE, Christoph, A-2700 Wiener Neustadt (AT); LINGNAU, Karen, A-1130 Vienna (AT); ZAUNER, Wolfgang, A-1030 Vienna (AT); ZETTLMEISSL, Gerd, A-1070 Vienna (AT)
(74) Representative: Sonn & Partner Patentanwälte
(86) International application number: PCT/EP2004/007540
(87) International publication number: WO 2005/004910

(56) References cited:
- WO-A1-01/21189
- WO-A1-02/32451
- WO-A2-2004/014936
- US-A- 5 683 864
- INCHAUSPE G ET AL: "DEVELOPMENT OF A HEPATITIS C VIRUS VACCINE" CLINICS IN LIVER DISEASE, SAUNDERS, PHILADELPHIA, PA, US, vol. 7, no. 1, 2003, pages 243-259, XP008039709 ISSN: 1089-3261
- LECHMANN MARTIN ET AL: "Vaccine development for hepatitis C" SEMINARS IN LIVER DISEASE, vol. 20, no. 2, 2000, pages 211-226, XP002316999 ISSN: 0272-8087

## Description

The present invention relates to HCV vaccines.

The immune system is a complex network of inter-related cell types and molecules, which has evolved in order to protect multicellular organisms from infectious microorganisms. It can be divided into the evolutionary older innate (or natural) immunity and adaptive (or acquired) immunity. The innate immune system recognises patterns, which are usually common and essential for pathogens. For this limited number of molecular structures germ-line encoded receptors have evolved. By contrast, cells of the adaptive immune system - B and T lymphocytes - can recognise a huge variety of antigenic structures. The receptors, termed according to the cell types expressing them, B cell receptor (BCR, its soluble versions are called antibodies) and T cell receptor (TCR, only cell-surface associated forms)27 are generated by somatic recombination and show a clonal distribution. Thus, initially there is only small number of cells with a certain specificity. Upon antigen encounter these cells start to divide (clonal expansion) to generate an effector population able to cope with the antigen. After elimination of antigen a specialised sub-population of cells specifically recognising this antigen remains as immunological memory. Taken together the adaptive immune system is slow (compared to innate immunity), however specific and it improves upon repeated exposure to a given pathogen/antigen.

T cells have a central role in adaptive immunity. Their receptors (TCRs) recognise "major histocompatibility complex" (MHC or HLA):peptide complexes on the surface of cells. These peptides are called T cell epitopes and represent degradation products of antigens. There are two major classes of T cells: CD8-positive cytotoxic T cells (CTL) are restricted to MHC class I. CD4-positive helper T cells (HTL) are restricted to MHC class II. HTL are essential for many features of adaptive immunity: activation of so called "professional antigen-presenting cells" (APCs), immunoglobulin (Ig) class switch, the germinal center reaction and Ig affinity maturation, activation of CTL, immunological memory, regulation of the immune response and others.

MHC molecules collect peptides inside the cell and present them on the cell surface to TCRs of T cells. There are two major classes of MHC, class I recognised by CD8-positive CTL and class II recognised by CD4-positive HTL.

MHC class I molecules consist of a membrane-anchored alpha-chain of 45 kDa and the non-covalently attached b2-microglobulin (b2m) of 12 kDA. Resolution of the 3-dimensional structure by X-ray crystallography (Stern and Wiley 1994) revealed that the alpha-chain possesses a cleft, which is closed at both ends and accommodates peptides from 8 to 11 amino acids length. Class I molecules are ubiquitously expressed, and the peptides they present originate from cytoplasmic proteins. These are degraded by the proteasome, and the resulting peptides are actively transported into the endoplasmatic reticulum (ER). There, with the help of several chaperones, MHC:peptide complexes are formed and transported to the cell surface (Heemels 1995). Thus, MHC class I mirrors the proteome of a cell on its surface and allows T cells to recognise intracellular pathogens or malignant cells.

MHC class II molecules consist of two membrane-anchored proteins (alpha- and beta-chain) of 35 kDa and 30 kDa, respectively. These together form a cleft, open at both ends, which can accommodate peptides of variable length, usually from 12 to 25 amino acids. Despite these differences, class I and II molecules share surprising structural similarity (Stern and Wiley 1994). Class II molecules are only expressed on professional APC including dendritic cells (DC), B-cells and macrophages/monocytes. These cells are specialised in taking up and processing antigens in the endosomal pathway. Immediately after their biosynthesis, class II molecules are complexed by the so-called invariant chain (Ii), which prevents binding of peptides in the ER. When vesicles containing class II:Ii complexes fuse with endosomes containing degradation products of exogenous antigen, Ii is degraded until the MHC binding cleft is only complexed by the so-called CLIP peptide. The latter is with the help of chaperones like HLA-DM exchanged by antigenic peptides (Villadangos 2000). Finally, MHC:peptide complexes are again presented on the surface of APCs, which interact in numerous ways with HTL.

Being both polygenic and extremely polymorphic, the MHC system is highly complex. For the class I alpha-chain in humans there are three gene loci termed HLA-A, -B and -C. Likewise, there are three class II alpha-chain loci (DRA, DQA, DPA); for class II beta-chain loci the situation is even more complex as there are four different DR beta-chains (DRB1,2,3,5) plus DQB and DPB. Except the monomorphic DR alpha-chain DRA, each gene locus is present in many different alleles (dozens to hundreds) in the population (Klein 1986). Different alleles have largely distinct binding specificities for peptides. Alleles are designated, for example, HLA-A*0201 or HLA-DRB1*0401 or HLA-DPA*0101/DPB*0401.

T cell epitopes have been identified by a variety of approaches (Van den Eynde 1997). T cell lines and clones have for instance been used to screen cDNA expression libraries for instance in the context of COS cells transfected with the appropriate HLA-molecule. Alternatively, biochemical approaches have been pursued. The latter involved elution of natural ligands from MHC molecules on the surface of target cells, the separation of these peptides by several chromatography steps, analysis of their reactivity with lymphocytes in epitope reconstitution assays and sequencing by mass spectrometry (Wölfel et al. 1994, Cox et al. 1994).

Recently the advent of highly sensitive cytokine detection assays like the IFN-gamma ELIspot allowed using lymphocytes directly ex vivo for screening of overlapping synthetic peptides (Maecker 2001, Kern 2000, Tobery 2001). Primarily, Kern et al. (1999&2000) used arrays of pools of overlapping 9mer peptides to map CD8+ T cell epitopes in vitro. Later, Tobery et al., 2001 modified this approach and demonstrated that pools containing as many as 64 20mer peptides may be used to screen for both CD8+ and CD4+ T cell epitopes in mice. Both these methods were based on the monitoring of antigen-specific response by measuring IFN-gamma production either by intracellular staining (Kern et al 2000) or in ELIspot assay (Tobery et al., 2001). By use of mixtures of 15-mers the CD4+ T cell responses are approximately equal to those detected when whole soluble protein was used as an antigen, while -not surprising- the CD8+ T cell responses are significantly higher than the often negligible responses detected with soluble protein stimulation. Furthermore, the CD8+ T cell responses to a mixture of 15 amino acid peptides are similar to those obtained with a mix of 8-12 amino acid peptides, selected to represent known MHC class I minimal epitopes. Most probably peptidases associated with the cell membrane are responsible for "clipping" peptides to optimal length under these circumstances (Maecker et al, 2001).

An interesting alternative is to screen synthetic combinatorial peptide libraries with specific lymphocytes. For instance, a decapeptide library consisting of 200 mixtures arranged in a positional scanning format, has been successfully used for identification of peptide ligands that stimulate clonotypic populations of T cells (Wilson, et al., J. Immunol., 1999, 163:6424-6434).

Many T cell epitopes have been identified by so called "Reverse immunological approaches" Rammensee 1999). In this case the protein giving rise to a potential T cell epitope is known, and its primary sequence is scanned for HLA binding motifs. Typically dozens to hundreds of candidate peptides or even a full set of overlapping peptides are synthesised and tested for binding to HLA molecules. Usually, the best binders are selected for further characterisation with regard to their reactivity with T cells. This can for instance be done by priming T cells in vitro or in vivo with the help of HLA transgenic mice.

Hepatitis C Virus (HCV) is a member of the flaviviridiae chronically infecting about 170 million people worldwide. There are at least 6 HCV genotypes and more than 50 subtypes have been described. In America, Europe and Japan genotypes 1, 2 and 3 are most common. The geographic distribution of HCV genotypes varies greatly with genotype 1a being predominant in the USA and parts of Western Europe, whereas 1b predominates in Southern and Central Europe (Bellentani 2000).

HCV is transmitted through the parenteral or percutan route, and replicates in hepatocytes. About 15% of patients experience acute self-limited hepatitis associated with viral clearance and recovery. About 80% of infected persons become chronic carriers. Infection often persists asymptomatically with slow progression for years, however ultimately HCV is a major cause of cirrhosis, end-stage liver disease and liver cancer (Liang 2000). Strength and quality of both HTL and CTL responses determine whether patients recover (spontaneously or as a consequence of therapy) or develop chronic infection (Liang 2000).

Standard therapy of HCV comprises a combination of pegylated interferon-alpha and the antiviral ribavirin. Virologic responses are, depending on the genotype, achieved in about 50% of HCV patients. The low tolerability and the considerable side effects of this therapy clearly necessitate novel therapeutic intervention including therapeutic vaccines (Cornberg 2002). However, presently the detailed understanding of which epitopes in which MHC combination lead to successful immune responses is lacking (Ward 2002). Therefore, a comprehensive analysis of the T-cell response against the entire HCV is required for development of therapeutic epitope-based vaccines.

The HCV virion contains a 9.5-kilobase positive single-strand RNA genome encoding a large single polyprotein of about 3000 amino acids. The latter is processed to at least 10 proteins by both host and HCV-enoded proteolytic activities (Liang 2000). Importantly, the HCV RNA-dependent RNA polymerase is error prone giving rise to the evolution of viral quasispecies and contributing to immune-escape variants (Farci 2000).

WO 01/21189A discloses mechanismus by which an antigen is recognized by T-cells to identify and prepare HCV epitopes in order to develop epitope-based vaccines directed towards HCV. US 5,683,864 A discloses combinations of HCV antigens consisting of an antigen from the C domain of HCV polyprotein and at least one additional antigens from either the NS3-domain, the NS4-domain, the S-domain or the NS5-domain.

Although many proposals have been made in the art in the last 15 years and many promising antigen candidates have been proposed during this time, there is still no HCV vaccine on the market (nor a satisfactory therapeutic treatment; the only available treatment, as mentioned above a combination of alpha interferon and ribavirin, is efficacious only in a minority of patients). Early after the publication of the HCV sequences, a myriad of epitopes and antigens were suggested as effective means for combatting HCV disease (e.g.: Koziel et al., J. Virol. 67(12), 7522-7532; Shirai et al., J. Virol. 68(5)(1994), 3334-3342; Leroux-Roels et al., Hepatology 23(1)(1996), 8-16; Hoffmann et al., Hepatology 21(3)(1995), 632-638; Sarobe et al., J. Clin. Invest. 102(6)(1998), 1239-1248; Battegay et al., J. Virol. 69(4)(1995), 2462-2470; Wentworth et al., Int. Imunol. 8(5)(1996), 651-659; Rehermann et al., J. Virol. 70(10)(1996), 7092-7102; Diepolder et al., J. Virol. 71(8)(1997), 6011-6019; Lamonaca et al., Hepatology 30(4)(1999), 1088-1098; WO00/11186, WO95/12766, WO95/27733, WO94/20127, WO95/25122, WO95/22317, WO94/25601, WO92/03458 and WO93/00365). Additionally, important proof-of-principle ex vivo neutralisation studies have been undertaken with chimpanzees, the other species other than homo sapiens that is susceptible to chronic HCV infection. Indeed, the study of immune responses induced in individuals and chimpanzees with acute resolved versus chronic infections has so far revealed only an emerging picture of potential protective immune responses following the establishment of active viral infection. However, the correlates of prophylactic vaccine protection following parenteral immunisation are expected to be largely extra-hepathic prior to HCV exposure. Only a limited number of vaccine efficacy studies have been performed with chimpanzees and these studies showed - at best - a protection of 5 out of 7 animals from infection after a low-dose homologous challenge with exactly the same clone being used for immunisation and challenge. In addition, HCV presents a high degree of variability which provides HCV with the capacity to escape vaccine- or infection-induced immune responses. In consideration of this problem, also a combination of different large protein fragments as a DNA vaccine has been suggested as alternative vaccination strategy (Rollier et al., J. Virol. 78(1)(2004), 187-196; Dunas-Carrera et al., Biotech. Appl. Biochem. Imm. Publ. (29.September 2003; BA20030112)).

Despite all the progress that has been made in the last 15, especially in the last 10 years there is still a pressing need to develop new therapies and vaccination strategies for HCV (Heile et al., J. Virol. 74(15)(2000), 6885-6892).

It is therefore an object of the present invention to provide such effective therapies and vaccination strategies for HCV.

The present invention therefore provides a Hepatitis C virus (HCV) vaccine as claimed in claim 1. Preferred embodiments of this HCV vaccine are claimed in claims 2 to 17. The vaccine of the present invention can be used for the preparation of a medicament for the prevention and treatment of an infection with HCV. A method for the preparation of this vaccine is claimed in claim 19.

In the application WO04/024182, filed 27 August 2003 (claiming a priority A 1367/2002 of 13 September 2002), a novel method for isolating HCV peptides useful in vaccination, especially HCV T cell epitopes, is described. In this application the concept of "HCV hotspot epitopes" was disclosed for the first time: a T-cell epitope "hotspot" is defined as a short peptide sequence at least comprising more than one T-cell epitope. For example, two or more epitopes may be located shortly after each other (shortly being defined as less than 5-10 amino acids), or directly after each other, or partially or even fully over-lapping. Hotspots may contain only class I or class II epitopes, or a combination of both. Epitopes in hotspots may have different HLA restrictions.

Due to the highly complex and selective pathways of class I and class II antigen processing, referred to in the introduction, T-cell epitopes cannot be easily predicted within the sequence of a polypeptide. Though widely used, computer algorithms for T-cell epitope prediction have a high rate of both false-negatives and false-positives.

Thus, as even individual T-cell epitopes are not obvious within the sequence of a polypeptide, the same is even more the case for hotspots. Several radically different experimental approaches are combined according to the present invention for T-cell epitope identification, including epitope capture, HLA-transgenic animals and *in vitro* stimulation of human mononuclear cells. All three approaches are systematically applied on over-lapping peptides spanning the antigen of interest, enabling comprehensive identification of epitopes. Upon such a comprehensive analysis, not limited to a particular HLA allele, but rather unravelling all possibly targeted epitopes within a population, epitope hotspots may become apparent. Within an antigen, only few if any sequences show characteristics of hotspots. Thus the identification of a hotspot is always a surprising event.

T-cell epitope hotspots offer important advantages: Hotspots can activate and can be recognised by different T-cell clones of a subject. Hotspots (when comprising epitopes with different HLA restriction) can interact with T-cells from different non HLA-matched individuals.

Epitope-based vaccines, so far have aimed at selected prevalent HLA-alleles, for instance HLA-A2, which is expressed in about half of Caucasians. Since other alleles are less frequent, epitope-based vaccines with broad worldwide population coverage will have to comprise many different epitopes. The number of chemical entities (for instance peptides) of a vaccine is limited by constraints originating from manufacturing, formulation and product stability.

Hotspots enable such epitope-based vaccines with broad worldwide population coverage, as they provide a potentially high number of epitopes by a limited number of peptides. Indeed, the prior art (see the aforementioned references concerning the epitopes discussed in the art and the fact that a HCV vaccine has not been developed yet) as it currently stands has clearly shown that the development of HCV vaccines has been regarded as problematic and that there has been a general failure to develop HCV vaccines and therapies despite the abundance of references through the last 10 years teaching the efficacy of HCV antigens in eliciting humoral and cellular immune response in several infection models. The treatment and prevention of HCV infection is wrought with complexity and severe unpredictability.

The concept of the present invention by providing HCV vaccines based on the HCV hotspot epitope characterisation now enables for the first time a HCV vaccination system enabling both, a suitable and specific prophylactic vaccination and an efficient treatment regimen for HCV infected patients, especially chronically infected patients.

It could be shown by the inventors of the present application that the use of only one or two specific epitopes in the HCV vaccine is not sufficient for satisfactory results in vaccination. Also the concept of providing a mixture of HCV proteins (Core, E1 and E2 as a DNA vaccine; Duenas-Carrera et al. (2003)) or a mixture of large fragments of such HCV proteins (Core, E1, E2 and NS3 as DNA vaccines; Rollier et al., (2004)) did not bring the desired results.

The HCV vaccines according to the present invention contain short polypeptides containing epitopes from the hotspot epitopes as listed above. The present vaccines are not dependent on the risks and uncertainties with respect to DNA vaccines (lack of long lasting immune response; largely extrahepatic action; mode of action, localisation, etc.). Moreover the present invention also does not face the severe manufacturing problems with the production of full proteins or long-chained protein fragments.

The hotspot epitopes disclosed herein have been identified via functional assays using primarily a Th1/Tc1-type reaction (i.e. interferon gamma) as read-out. It is well known that (whole or at least large fragment) antigens do not only contain such agonist epitopes, but may also encode antagonist ligands. Such antagonists potentially present in whole antigens, but absent in hotspots may impede immunogenicity and efficacy of the vaccine. Moreover, whole antigens will almost certainly induce a humoral immune response. Such an antibody response has per se little effect on HCV residing within cells, but it can severely compromise the desired T-cell response. The epitopes being derived from hotspots according to the present invention, due to their restricted length, have a much lower probability to induce unwanted antibody responses. Finally, only relatively small parts of HCV antigens are conserved between different genotypes. Thus, whole antigens used in a vaccine may induce responses against non-conserved regions or epitopes, irrelevant for most of the patients. In contrast, the hotspots disclosed herein are derived from regions conserved > 80% among the major HCV genotypes 1, 2 and 3.

HLA-super types (as disclosed in e.g. WO 01/21189) describe the fact that in some cases a given HLA-class I T-cell epitope is not exclusively restricted to on and only one HLA-allele but can also be recognised by T-cells in context of another HLA-allele (example: a peptide may bind to HLA-B*0702 and -B*3501). Such peptides by nature have broader HLA coverage in the population and are therefore interesting vaccine candidates.

In contrast a T-cell epitope hotspot according to the present invention significantly differs from such a HLA-super type, because a hotspot according to the present invention is a peptide region within a protein that shows an unusual high number of different adjacent and even overlapping epitopes. Hotspot deliver a high number of epitopes in relatively short regions, at the same time the proper processing of the epitopes within hotspots is guaranteed.

Indeed, the hotspots according to the present invention show a remarkably low variation between different HCV species which makes them even more favourable for vaccine approaches, because also heterologous challenge is possible even though only small polypeptides are used. Preferably the length of the epitopes according to the present invention is at least 6, 7, 8, 9, 10, 11 or 12 amino acids.

According to the present invention the epitopes for a given vaccine may be selected from the hotspot regions as defined above. Of course, either the whole hotspot region may be provided in the vaccine or only parts thereof (as long as the part contains at least one epitope). The epitopes to be used according to the present invention may also be sequence variants having deletions (of preferably 1, 2, 3, 4, 5, 6 or 7 amino acids) in the hotspot "master" sequence as defined above. Preferably, the deletions inside the hotspot sequence do not alter the epitopes; of course at least one epitope in the hotspot region has to be preserved despite the deletion.

The epitopes may be provided as polypeptides without any modifications; modifying the polypeptide chain with certain chemical or biological moieties may be appropriate, especially with respect to solubilising or pharmaceutical formulation considerations (e.g. as described in WO01/78767). The epitopes used in the present HCV vaccine may either comprise the whole hotspot region or a contiguous fragment thereof (containing at least one of the epitopes contained in the hotspot) or comprise a non-contiguous part of the hotspot (however, also containing at least one of the epitopes contained in the hotspot; in such a case one or more linkers (e.g. amino acid or peptide linkers) can be provided between the non-contiguous parts of this hotspot).

According to a preferred embodiment, the HCV vaccine according to the present invention comprises at least three, especially at least four epitopes, each from a different hotspot epitope.

Even more preferred, the HCV vaccine is comprising at least five epitopes, each from a different hotspot epitope. Although the higher the number of epitopes is in such an epitope-based vaccin, the larger the problems with respect to formulation are, a number of 4, 5 or 6 epitopes is the optimal balance between broad range of protection capacities and formulation drawbacks, especially with respect to production on an industrial scale.

In certain cases therefore also a HCV vaccine comprising at least six epitopes, each from a different hotspot epitope may be a preferred embodiment of the present invention. The optimal number of hotspot epitopes is also dependent on the relative interaction of the selected epitopes with each other which has proven to be the major obstacle for certain combinations of short lenght polypeptides in a vaccine.

Preferred HCV vaccines according to the present invention are **characterised in that** - in addition to the epitopes a), b) and c) of claim 1 - the further epitopes are selected from the following groups:
KFPGGGQIVGGVYLLPRRGPRLGVRATRK, KFPGGGQIVGGVYLLPRRGPRL, YLLPRRGPRL, LPRRGPRL, GPRLGVRAT, RLGVRATRK;
GYKVLVLNPSVAAT, AYAAQGYKVL, AYAAQGYKVLVLNPSVAAT; DLMGYIPAV, GYIPLVGAPL, DLMGYIPLVGAPL;
CINGVCWTV, GEVQVVSTATQSFLAT, GEVQVVSTATQSFLATCINGVCWTV;
HMWNFISGIQYLAGLSTLPGNPA, MWNFISGIQYLAGLSTLPGN, NFISGIQY-LAGLSTLPGNPA, QYLAGLSTL, HMWNFISGI;
VDYPYRLWHYPCTVNFTIFKVRMYVGGVEHRL, DYPYRLWHYPCTVNFTIFKI, DYPYRLWHYPCTVNFTIFKV, VDYPYRLWHYPCTVNYTIFKIRMYVGGVEHRL, DYPYRLWHYPCTVNYTIFKI, DYPYRLWHY, TVNYTIFKI, TINYTIFK, TVNFTIFKV, HYPCTVNYTI, HYPCTVNFTI, RMYVGGVEHR;
AAWYELTPAETTVRLR, TPAETTVRL;
GWRLLAPITAYSQQTRGLLGCIV, TAYSQQTRGLLGCIV, TAYSQQTRGLLG, GQGWRLLAPITAYSQ, RLLAPITAY, GQGWRLLAPITAYSQQTRGLLGCIV, GQGWRLLAPITAYSQQTRGLLG, AYSQQTRGLL, AYSQQTRGL; IGLGKVLVDILAGYGAGVAGALVAFK, ILAGYGAGV, VAGALVAFK, GYGAGVAGAL;
VVCCSMSYTWTGALITPC, SMSYTWTGALITP, SMSYTWTGAL, SYTWTGALI; FTDNSSPPAVPQTFQV;
LEDRDRSELSPLLLSTTEW, LEDRDRSELSPLLLST, RSELSPLLL, ELSPLLLST, DRDRSELSPL, LEDRDRSEL, LEDRDRSEL;
YLVAYQATVCARAQAPPPSWD, YLVAYQATV;
MSTNPKPQRKTKRNTNR, PQRKTKRNTNR, QRKTKRNTN, RKTKRNTNR, MSTNPKPQR, MSTNPKPQK;
LINTNGSWHINRTALNCNDSL, NGSWHINRTALNCNDSL, LINTNGSWHI, RTALNCNDSL, LINTNGSWHINRTALN, SWHINRTALN;
TTILGIGTVLDQAET, TTILGIGTV, TILGIGTVL; FDSSVLCECYDAGAAWYE, FDSSVLCECYDAGCA, VLCECYDAGA, VVLCECYDA-GAAWYE;
ARLIVFPDLGVRVCEKMALY, ARLIVFPDL, RLIVFPDLGV, RVCEKMALY, AFCSAMYVGDLCGSV;
GVLFGLAYFSMVGNW;
TRVPYFVRAQGLIRA;
TTLLFNILGGWVAAQ, LLFNILGGWV.

Generally, from one group of epitopes only one epitope should be selected in order to achieve good results. Of course not all of the hotspot epitopes listed above are equally powerful and efficacy may vary between different population groups. Preferably, an individual vaccination strategy may be applied for each group which is mainly based on HLA coverage. Therefore, a preferred HCV vaccine according to the present invention is **characterised in that** it comprises at least the following hotspot epitopes:
KFPGGGQIVGGVYLLPRRGPRLGVRATRK,
AYAAQGYKVLVLNPSVAAT,
DLMGYIP(A/L)VGAPL,
GEVQVVSTATQSFLATCINGVCWTV and
HMWNFISGIQYLAGLSTLPGNPA.

As an alternative to the precise antigens and epitopes listed herein, antigen/epitope variants may be used in the vaccines according to the present invention. Preferred variants are designed according to homologues (containing one or more exchanges being present in homologous HCV strains) or heteroclictic epitopes. Among preferred variants are those that vary from a sequence disclosed herein by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe and Tyr.

Further particularly preferred in this regard are variants having the amino acid sequence of any polypeptide disclosed herein, in which 1, 2, 3, 4 or 5 amino acid residues are substituted, deleted or added, in any combination. Preferred among these are silent substitutions, additions and deletions, which do not alter the properties and activities of the polypeptide of the present invention. Especially preferred in this regard are conservative substitutions. Specifically suitable amino acid substitutions are those which are contained in homologues for the sequences disclosed herein. A suitable sequence variant of an antigen or epitope as disclosed herein therefore includes one or more variations being present in one or more strains or sero-types of HCV (preferably 1, 2, 3, 4 or 5 amino acid exchanges which are based on such homolog variations). Such antigens comprise sequences which may be naturally occurring sequences or newly created artificial sequences. These preferred antigen variants are based on such naturally occurring sequence variations, e.g. forming a "mixed sequence" for the antigenic regions of the polypeptides according to the present invention. For example, a given HCV sequence as disclosed herein may be amended by including such one or more variations thereby creating an artificial (i.e. non-naturally occurring) variant of this given (naturally occurring) antigen or epitope sequence.

It is clear that also epitopes or peptides derived from the present epitopes or peptides by amino acid exchanges improving, conserving or at least not significantly impeding the T cell activating capability of the epitopes are covered by the epitopes or peptides according to the present invention. Therefore, the present epitopes or peptides also cover epitopes or peptides, which do not contain the original sequence as derived from a specific strain of HCV, but trigger the same or preferably an improved T cell response. These epitopes are referred to as "heteroclitic". These include any epitope, which can trigger the same T cells as the original epitope and has preferably a more potent activation capacity of T cells preferably in vivo or also in vitro. Also the respective homologous epitopes from other strains of HCV are encompassed by the present invention.

Heteroclitic epitopes can be obtained by rational design i.e. taking into account the contribution of individual residues to binding to MHC/HLA as for instance described by Ramensee et al. 1999 (Immunogenetics 50: 213-219) or Sturniolo et al. 1999 (Nature Biotechnology 17: 555-562), combined with a systematic exchange of residues potentially interacting with the TCR and testing the resulting sequences with T cells directed against the original epitope. Such a design is possible for a skilled man in the art without much experimentation.

Another possibility includes the screening of peptide libraries with T cells directed against the original epitope. A preferred way is the positional scanning of synthetic peptide libraries. Such approaches have been described in detail for instance by Blake et al 1996 () and Hemmer et al. 1999 () and the references given therein.

As an alternative to epitopes represented by the cognate HCV derived amino acid sequence or heteroclitic epitopes, also substances mimicking these epitopes e.g. "peptidemimetica" or "retro-inverso-peptides" can be applied.

Further chemical groups, especially amino acid residues, linkers (and binding to carriers), transport facilitating groups, etc., may be added to the N- and/or C-terminus of the eptiopes in the HCV vaccine according to the present invention. Amino acid attachments as described in WO01/78767 are preferred.

Another aspect of the design of improved hotspot epitopes is their formulation or modification with substances increasing their capacity to stimulate T cells. These include T helper cell epitopes, lipids or liposomes (or the preferred modifications as described in WO 01/78767).

Another way to increase the T cell stimulating capacity of epitopes is their formulation with immune stimulating substances for instance antibodies, cytokines or chemokines like interleukin-2, -7, -12, -18, type I and II interferons (IFN), especially IFN-alpha, GM-CSF, TNF-alpha, flt3-ligand and others.

According to a further aspect, the present invention is drawn to the use of a HCV epitope or HCV peptide according to the present invention for the preparation of a HLA restricted vaccine for treating or preventing hepatitis C virus (HCV) infections.

Another preferred HCV vaccine according to the present invention comprises the following epitopes:
KFPGGGQIVGGVYLLPRRGPRLGVRATRK, DYPYRLWHYPCTVNFTIFKV AAWYELTPAETTVRLR, TAYSQQTRGLLG, HMWNFISGIQYLAGLSTLPGNPA, IGLGKVLVDILAGYGAGVAGALVAFK and SMSYTWTGALITP.

The present HCV vaccine preferably comprises at least one A2 epitope and at least one DR1 epitope.

The present HCV vaccine preferably comprises at least one DR7 epitope.

The following combination of epitopes is regarded as specifically powerful (at least one from the groups (1) to (5)):
(1) KFPGGGQIVGGVYLLPRRGPRLGVRATRK or KFPGGGQIVGGVYLLPRRGPRL or YLLPRRGPRLGVRATRK or YLLPRRGPRL or LPRRGPRL or, LPRRGPRLGVRATRK or GPRLGVRATRK or RLGVRATRK or KFPGGYLLPRRGPRLGVRATRK,
(2) AYAAQGYKVLVLNPSVAAT or AYAAQGYKVL or AAQGYKVLVLNPSVAAT or KVLVLNPSVAAT or GYKVLVLNPSVAAT or AYAAQGYKVLVLNPSV or AYAAQGYKVLVLNPSVAA or AAQGYKVLVLNPSVA or AYAAQGYKVLPSVAAT or AYAAQGYKVLAAT,
(3) DLMGYIP(A/L)VGAPL or DLMGYIPALVGAPL or DLMGYIP(A/L)VG or DLMGYIP(A/L)VGAP or DLMGYIP(A/L)V or DLMGYIPLVGAPL or DLMGY-IPLVGA or DLMGYIPLV,
(4) GEVQVVSTATQSFLATCINGVCWTV or GEVQVVSTATQSFLAT or CINGVCWTV or VSTATQSFLATCINGVCWTV or TQSFLATCINGVCWTV or GEVQVVSTATQSFLAT-CING or GEVQVVSTATQSFLAT,
(5) HMWNFISGIQYLAGLSTLPGNPA or MWNFISGIQYLAGLSTLPGNPA or HMWNFISGI or MWNFISGIQYLAGLSTLPGN or NFISGIQYLAGLSTLPGN or QYLAGLSTL or HMWNFISGIQYLAGLSTL or HMWNFISGISTLPGNPA or HMWQY-LAGLSTLPGNPA or MWNFISGIQYLAGLSTLPGN; especially a HCV vaccine comprising the epitopes GYKVLVLNPSVAAT, DLMGYIPAV, CINGVCWTV and HMWNFISGIQYLAGLSTLPGNPA has been proven to be specifically powerful.

It is preferred to provide specifically suitable auxiliary substances for the present HCV vaccines. Therefore certain types of immunostimulating substances have proven to be advantageous for the present invention.

Therefore, a preferred HCV vaccine further contains a peptide comprising a sequence R₁-XZXZ_{N}XZX-R₂, whereby N is a whole number between 3 and 7, preferably 5, X is a positively charged natural and/or non-natural amino acid residue, Z is an amino acid residue selected from the group consisting of L, V, I, F and/or W, and R₁ and R₂ are selected independantly one from the other from the group consisting of -H, -NH₂, -COCH₃, -COH, a peptide with up to 20 amino acid residues or a peptide reactive group or a peptide linker with or without a peptide; X-R₂ may be an amide, ester or thioester of the C-terminal amino acid residue of the peptide ("Peptide A").

Another preferred embodiment of the HCV vaccine further contains an immunostimulatory oligodeoxynucleic acid molecule (ODN) having the structure according to the formula (I) 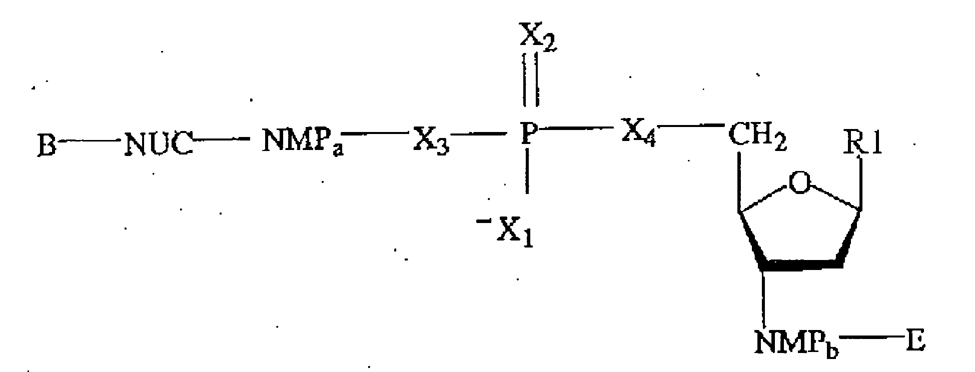 wherein
R1 is selected from hypoxanthine and uracile, any X is O or S,
any NMP is a 2' deoxynucleoside monophosphate or monothiophosphate, selected from the group consisting of deoxyadenosine-, deoxyguanosine-, deoxyinosine-, deoxycytosine-, deoxyuridine-, deoxythymidine-, 2-methyl-deoxyinosine-, 5-methyl-deoxycytosine-, deoxypseudouridine-, deoxyribosepurine-, 2-amino-deoxyribosepurine-, 6-S-deoxyguanine-, 2-dimethyl-deoxyguanosine- or N-isopentenyl-deoxyadenosine-monophosphate or -monothiophosphate,
NUC is a 2' deoxynucleoside, selected from the group consisting of deoxyadenosine-, deoxyguanosine-, deoxyinosine-, deoxycytosine-, deoxyinosine-, deoxythymidine-, 2-methyl-deoxyuridine-, 5-methyl-deoxycytosine-, deoxypseudouridine-, deoxyribosepurine-, 2-amino-deoxyribosepurine-, 6-S-deoxyguanine-, 2-dimethyl-deoxyguanosine- or N-isopentenyl-deoxyadenosine,
a and b are integers from 0 to 100 with the proviso that a + b is between 4 and 150, and
B and E are common groups for 5' or 3' ends of nucleic acid molecules ("I-/U-ODN").

A HCV vaccine composition according to the present invention may further comprise a polycationic compound, preferably a polycationic polymer, more preferably a polycationic peptide, especially polyarginine, polylysine or an antimicrobial peptide.

The polycationic compound(s) to be used according to the present invention may be any polycationic compound which shows the characteristic effect according to the WO 97/30721. Preferred polycationic compounds are selected from basic polypeptides, organic polycations, basic polyaminoacids or mixtures thereof. These polyaminoacids should have a chain length of at least 4 amino acid residues. Especially preferred are substances containing peptidic bonds, like polylysine, polyarginine and polypeptides containing more than 20%, especially more than 50% of basic amino acids in a range of more than 8, especially more than 20, amino acid residues or mixtures thereof. Other preferred polycations and their pharmaceutical compositions are described in WO 97/30721 (e.g. polyethyleneimine) and WO 99/38528. Preferably these polypeptides contain between 20 and 500 amino acid residues, especially between 30 and 200 residues. These polycationic compounds may be produced chemically or recombinantly or may be derived from natural sources.

Cationic (poly)peptides may also be polycationic anti-bacterial microbial peptides. These (poly)peptides may be of prokaryotic or animal or plant origin or may be produced chemically or recombinantly. Peptides may also belong to the class of defensins. Such host defense peptides or defensins are also a preferred form of the polycationic polymer according to the present invention. Generally, a compound allowing as an end product activation (or down-regulation) of the adaptive immune system, preferably mediated by APCs (including dendritic cells) is used as polycationic polymer.

Polycationic substances used in the method according to the present invention are cathelicidin derived antimicrobial peptides or derivatives thereof (WO02/13857 A), especially antimicrobial peptides derived from mammalian cathelicidins, preferably from human, bovine or mouse, or neuroactive compounds, such as (human) growth hormone (as described e.g. in WO01/24822).

Polycationic compounds derived from natural sources include HIV-REV or HIV-TAT (derived cationic peptides, antennapedia peptides, chitosan or other derivatives of chitin) or other peptides derived from these peptides or proteins by biochemical or recombinant production. Other preferred polycationic compounds are cathelin or related or derived substances from cathelin, especially mouse, bovine or especially human cathelins and/or cathelicidins. Related or derived cathelin substances contain the whole or parts of the cathelin sequence with at least 15-20 amino acid residues. Derivations may include the substitution or modification of the natural amino acids by amino acids which are not among the 20 standard amino acids. Moreover, further cationic residues may be introduced into such cathelin molecules. These cathelin molecules are preferred to be combined with the HCV peptide composition according to the present invention. However, these cathelin molecules surprisingly have turned out to be also effective as an adjuvant for a antigen without the addition of further adjuvants. It is therefore possible to use such cathelin molecules as efficient adjuvants in vaccine formulations with or without further immunactivating substances. Preferably, the present HCV vaccine further contains an Al(OH)₃ adjuvant and/or a polycationic peptide.

According to a specifically preferred embodiment, the above mentioned Peptide A is KLKL₅KLK and/or the above mentioned I-/U-ODN is oligo d(IC)₁₃.

In certain embodiments, it can be preferred that the HCV vaccine further contains an oligodeoxynucleotide containing a CpG-motif.

Preferably, the HCV vaccine according to the present invention is lyophilised in a form which is reconstitutable within 15 min. at 37°C. Depending on the individual epitopes in the HCV vaccine, this may be a difficult task. Therefore, the present invention also provides for efficient means for formulating such a HCV vaccine.

The HCV vaccine according to the present invention preferably contains between 10 µg and 10 mg of each epitope (per dose).

Depending on the application the number of the individual peptides and especially their concentration in the mixture varies. In a preferred embodiment of the present invention the concentration of the individual peptides in the HCV vaccine to be delivered to the patient ranges from 5µg/ml to 5mg/ml, preferably from 50µg/ml to 4mg/ml, more preferably from 100µg/ml to 3mg/ml.

The lyophilised HCV vaccine according to the present invention preferably contains traces of acetic acid due to the lyophilisation process according to the present invention.

According to another aspect, the present invention also relates to the use of the present HCV vaccine for the preparation of a medicament for the prevention and treatment of an infection with HCV, i.e. administering an effective immunising dose of a HCV vaccine according to the present invention to an individual, especially a HCV patient or an individual being at risk of aquiring a HCV infection (e.g. hospital personnel, clinical research personnel, blood donation personnel, etc.).

Solubilising peptide mixtures such as the HCV vaccines according to the present invention can be difficult. It is therefore an aim of the present invention to provide methods for the solubilisation of the HCV peptide mixtures according to the present invention, i.e. peptide mixtures which are composed of two or more peptides, especially those consisting of hydrophilic as well hydrophobic peptides. Another aim is to make available a method for the manufacturing of sterile and optionally lyophilised pharmaceutical formulations containing peptide mixtures.

Therefore, the present invention provides according to another aspect a method for making a pharmaceutical preparation comprising the solubilisation of a HCV peptide mixture according to the present invention, **characterised in that** the peptide mixture is solubilised by an aqueous solution containing at least one organic acid selected from the group consisting of formic acid, acetic acid, propionic acid, butyric acid and halogenated or hydroxylated forms thereof. In order to elongate the shelf life the solubilised peptide mixture may be further sterilised (by filtration, irradiation, heat treatment, chemical sterilisation or other methods) and lyophilised.

In a preferred embodiment the peptide mixture contains hydrophilic as well hydrophobic peptides. Of course the present method is also usable for peptide mixtures containing only one type of peptides (specifically for mixtures containing e.g. two or more hydrophilic peptides).

In another preferred embodiment the peptides which are dissolved by an aqueous solution according to the present invention contain at least 6, preferably at least 8, more preferably at least 10, especially at least 12 amino acids. Peptides as well polypeptides containing a maximum number of 30, 40 or 50 (or even up to 100, although long epitopes are less preferred due to the disadvantages of longer antigen fragments) amino acids can be dissolved according to the present invention.

For this aspect of the present invention peptides are characterised by their solubility. Peptides which are soluble in an aqueous solution less than 100µg/ml are considered hydrophobic. On the other hand hydrophilic peptides dissolve in a buffered aqueous solution in concentration over 100µg/ml. Hydrophilic peptides may further be divided into cationic (>20% basic amino acids including lysine, arginine, histidine), anionic (>20% acidic amino acids including aspartic acid, glutamic acid) and hydroxyl-rich peptides (>30% -OH containing amino acids like serine, threonine, tyrosine).

According to another aspect, the present invention therefore relates to a method for the preparation of the present HCV vaccine which is characterised by the following steps:
- chemically synthesising the at least two epitopes as defined above,
- solubilising these epitopes by an aqueous solution containing at least one organic acid selected from the group consisting of formic acid, acetic acid, propionic acid, butyric acid and halogenated or hydroxylated forms thereof,
- mixing the solubilised epitopes and
- optionally lyophilising the mixed epitopes.

The HCV peptide mixture can be deep frozen (if no lyophilisation step is carried out) for storage reasons.

The peptides of the peptide mixture according to the present invention are synthesised by standard chemical methods (e.g. solid phase synthesis according to Merrifield). Of course the peptides may also be obtained by chemical or enzymatic fragmentation of recombinant produced or native isolated proteins. In another embodiment the HCV peptides are directly isolated from eukaryotic and prokaryotic cells. In all three cases additional purification of the peptide or polypeptide of interest will in some cases be required before they are lyophilised and mixed together.

It is a basic requirement for this specific method that the organic acids used in aqueous solutions for the solubilisation of peptides are pharmaceutically acceptable. In a preferred embodiment of the present invention the organic acids are acetic acid and/or formic acid containing optionally acetonitrile.

The experiments revealed, depending on the composition of the peptide mixture, that the concentration of the organic acid in the aqueous solution has to be at least 10%, preferably at least 20%, preferably at least 30%, preferably at least 40%, preferably at least 50%, preferably at least 60%.

In some cases the addition of derivatives of organic acids (e.g. acetonitrile) to the aqueous solution may be helpful to solubilise peptide mixtures containing a larger quantity of hydrophobic peptides. Also organic solvents such as DMSO and DMF contribute to an enhanced dissolution of peptide mixtures containing an increased concentration of hydrophobic peptides. However, aqueous peptide mixtures containing DMSO and/or DMF cannot be freeze-dried.

In a preferred embodiment bulking agents are added to the peptide mixture when the product is intended to be lyophilised. Especially at low peptide concentrations the forming of a good lyophilisation cake is not guaranteed. Therefore at low amounts of peptides bulking agents are added. Preferred bulking agents are sorbitol, mannitol, polyvinylpyrrolidone (PVP) and mixtures thereof.

In another preferred embodiment the solubilised peptide mixture is sterilised by filtration. In order to get an increased recovery of the product and to allow filtration of large amounts of the peptide mixture, the containing peptides have to be completely solubilised and no gel may be formed.

The solubilised and optionally sterilised peptide mixture can be lyophilised directly or after filling into vials. Lyophilisation is a useful and effective method to prolong the shelf life of peptide mixtures as described above. With the solubilised peptides a lyophilised preparation of a mixture of peptides, containing a maximum of 5% of residual solvent (water in aqueous systems) and traces of the organic acid, can be obtained which is reconstitutable in a buffered aqueous solution containing NaCl and/or sorbitol within 10 minutes of >95%, preferably of 98%, especially of 99%, resulting in a turbid suspension or clear solution (depending on the composition of the peptide mixture). Such a quick reconstitution is specifically necessary in emergency cases, where a ready-to-use solution has to be present within a few minutes with an almost complete re-solvation of the whole dosis of a lyophilised vial.

The invention will be described in more detail by the following examples and figures, but the invention is of course not limited thereto.

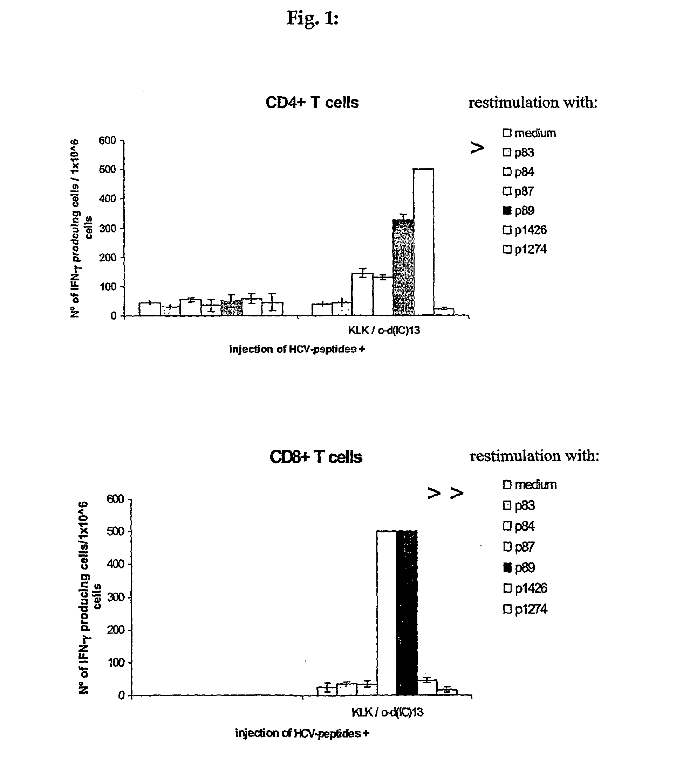

Fig. 1 shows that KLK/O-d(IC)₁₃ induces HCV-peptide specific type 1 cellular immune responses;

### EXAMPLES:

### Example I. Binding of HCV derived peptides to HLA class II molecules

According to the WO04/024182, several new peptides incorporating sequences from overlapping reactive HCV peptides or avoiding critical residues like cystein were synthesised. These were retested for their affinities to class II soluble HLA molecules, and results were compared to those obtained with the original (Table 1).

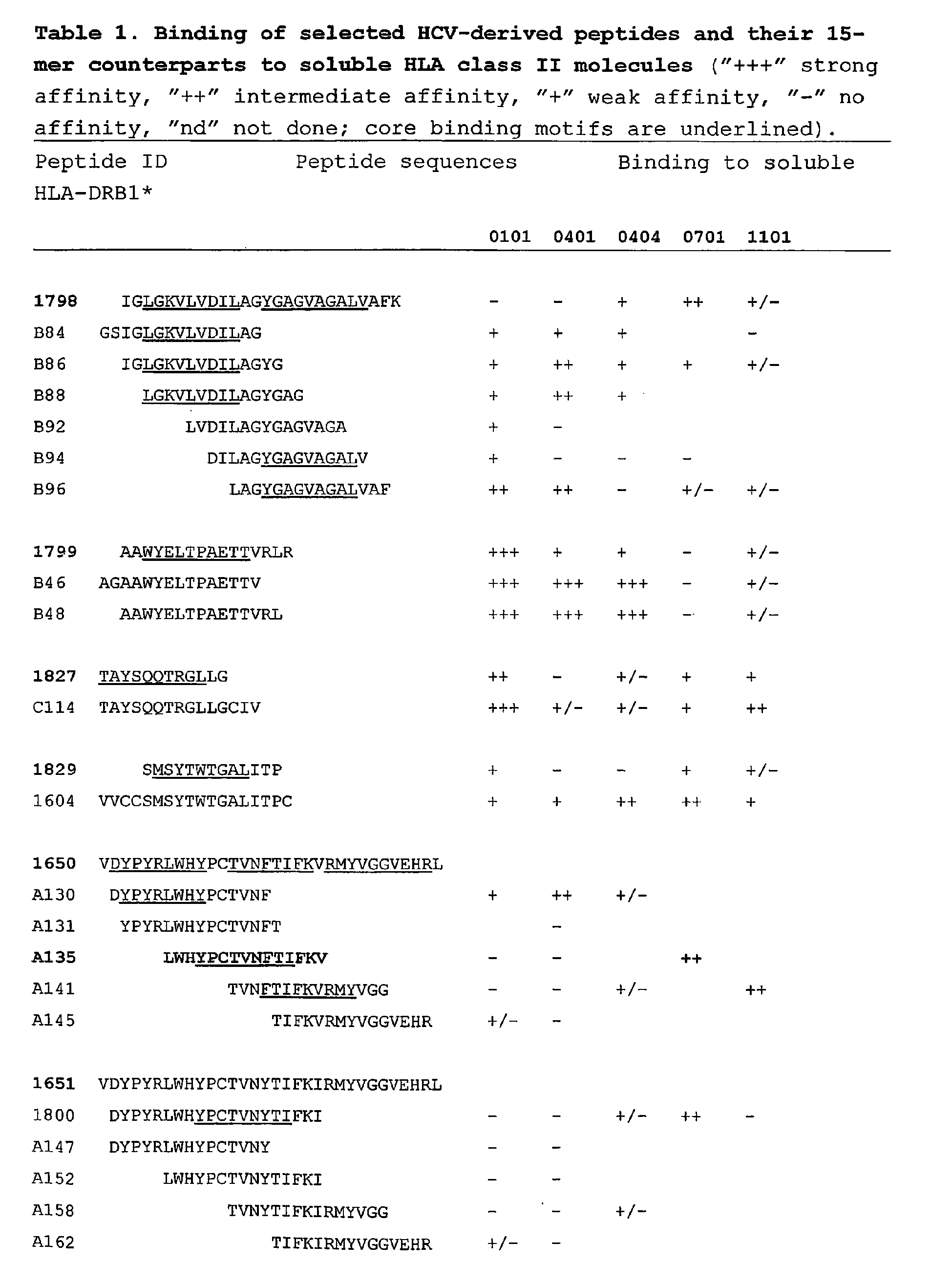

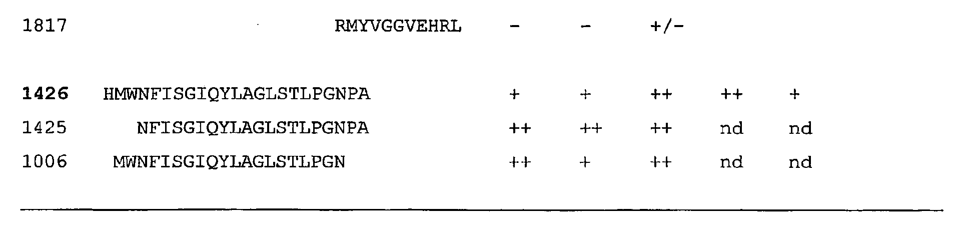

Abolished affinities to DRB1*0101 and DRB1*0401 molecules in the case of peptide 1798 in comparison with its shorter counterparts (B84 - B96) is probably due to the long sequence (26 amino acids) which can have a secondary structure that prevents binding. It is to be expected that in vivo, upon proteolytic cleavage, peptide 1798 will give rise to two shorter class II epitopes. Removed cystein (C) residues in peptides 1827 and 1829 (derivatives of peptides C114 and 1604, respectively) seem to be crucial for binding to DRB1*0401 molecules but do not essentially change affinities to other tested DR subtypes.

### Example II. Identification and Characterisation of HCV-epitope hotspots

As outlined above, a T-cell epitope hotspot (a "hotspot") is defined as a short peptide sequence at least comprising more than one T-cell epitope. For example, two or more epitopes may be located shortly after each other (shortly being defined as less than 5-10 amino acids), or directly after each other, or partially or even fully over-lapping. Hotspots may contain only class I or class II epitopes, or a combination of both. Epitopes in hotspots may have different HLA restrictions.

Due to the highly complex and selective pathways of class I and class II antigen processing, referred to in the introduction, T-cell epitopes cannot be easily predicted within the sequence of a polypeptide. Though widely used, computer algorithms for T-cell epitope prediction have a high rate of both false-negatives and false-positives.

Thus, as even individual T-cell epitopes are not obvious within the sequence of a polypeptide, the same is even more the case for hotspots. Several radically different experimental approaches are combined according to the present invention for T-cell epitope identification, including epitope capture, HLA-transgenic animals and *in vitro* stimulation of human mononuclear cells. All three approaches are systematically applied on over-lapping peptides spanning the antigen of interest, enabling comprehensive identification of epitopes. Upon such a comprehensive analysis, not limited to a particular HLA allele, but rather unravelling all possibly targeted epitopes within a population, epitope hotspots may become apparent. Within an antigen, only few if any sequences show characteristics of hotspots. Thus the identification of a hotspot is always a surprising event.

T-cell epitope hotspots offer important advantages: Hotspots can activate and can be recognised by different T-cell clones of a subject. Hotspots (when comprising epitopes with different HLA restriction) can interact with T-cells from different non HLA-matched individuals.

Epitope-based vaccines, so far have aimed at selected prevalent HLA-alleles, for instance HLA-A2, which is expressed in about half of Caucasians. Since other alleles are less frequent, epitope-based vaccines with broad worldwide population coverage will have to comprise many different epitopes. The number of chemical entities (for instance peptides) of a vaccine is limited by constraints originating from manufacturing, formulation and product stability.

Hotspots enable such epitope-based vaccines with broad worldwide population coverage, as they provide a potentially high number of epitopes by a limited number of peptides.

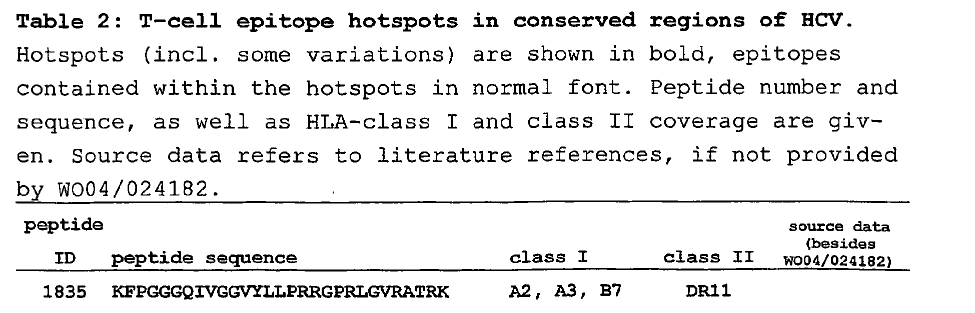

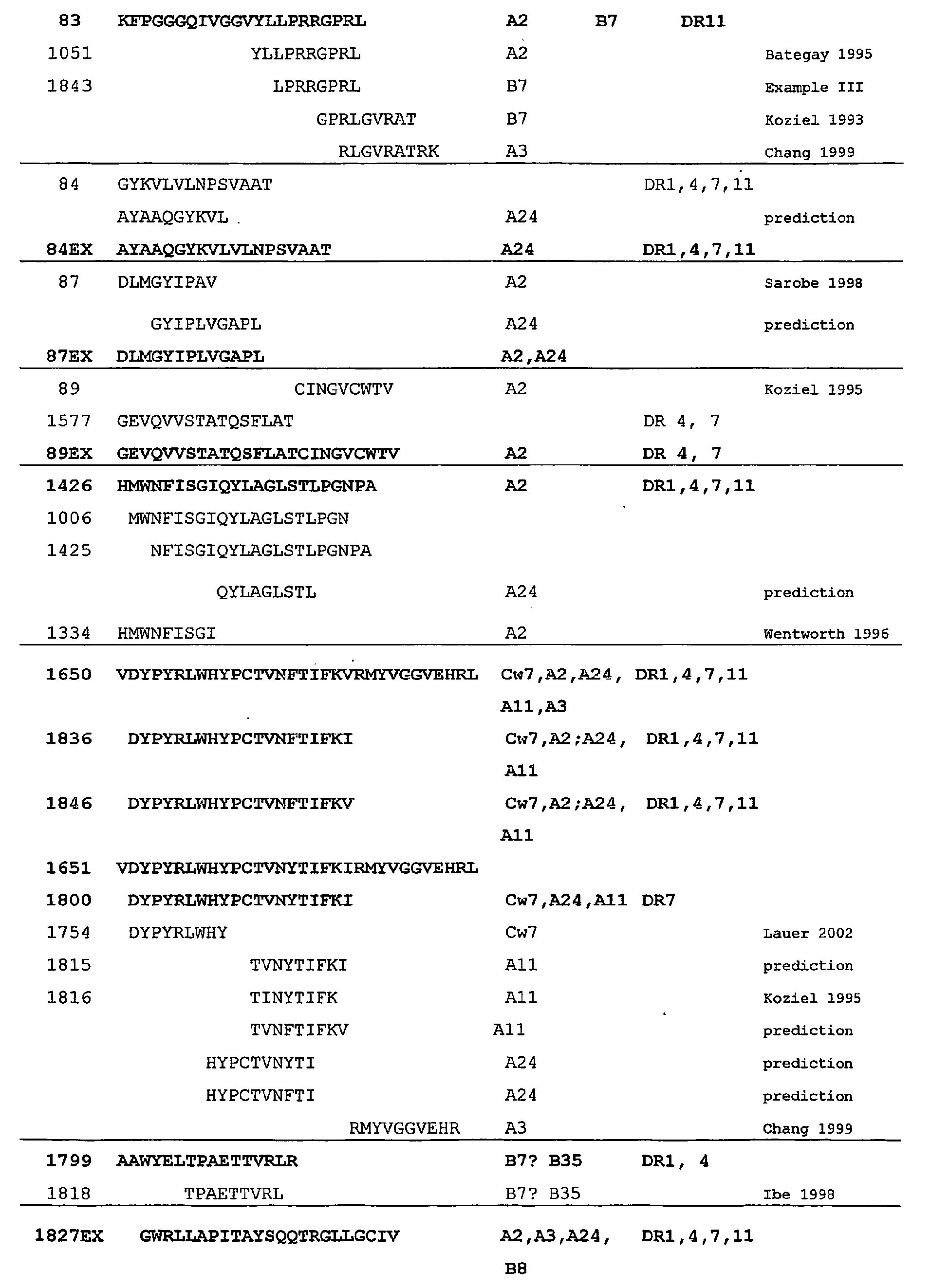

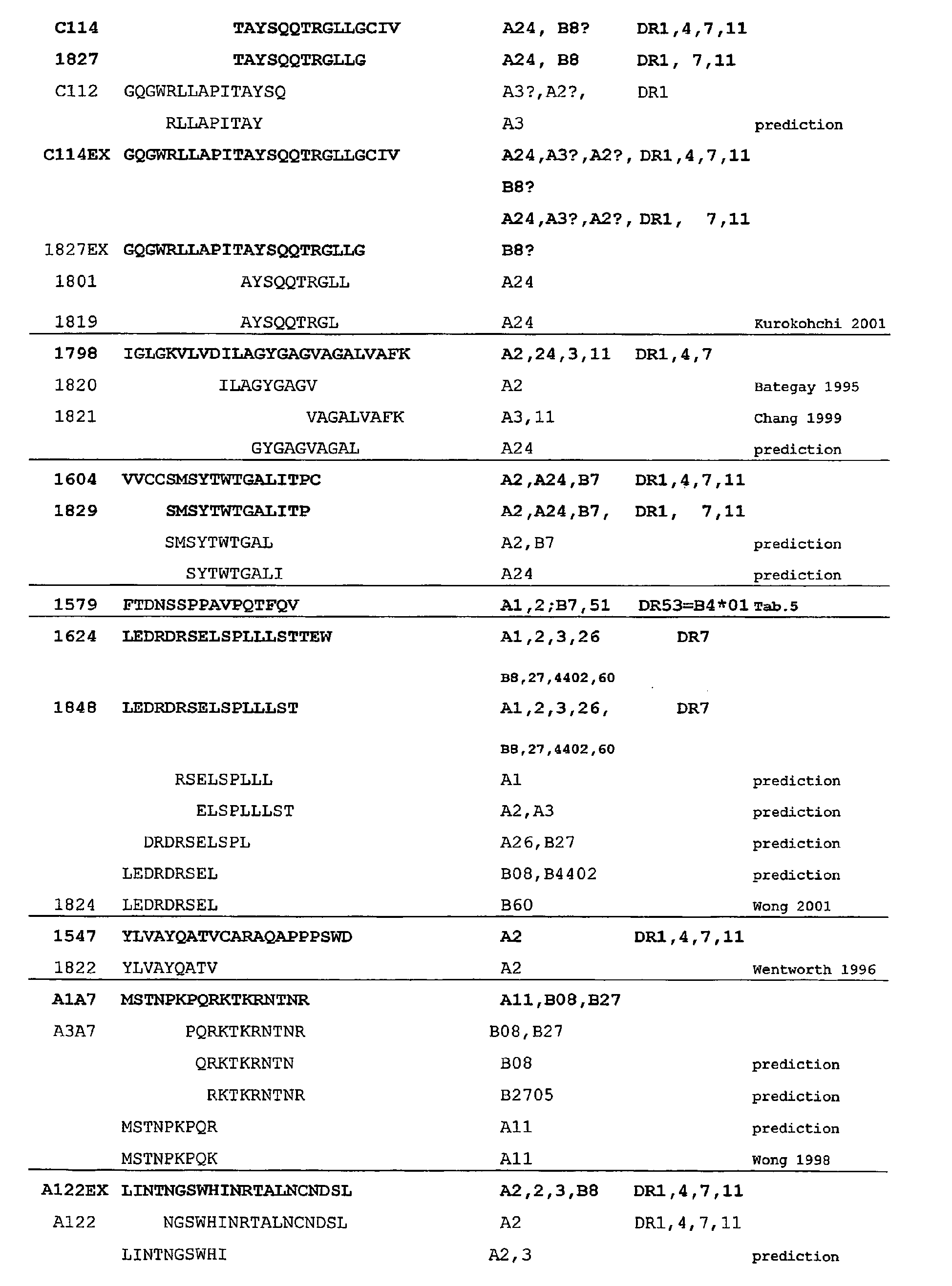

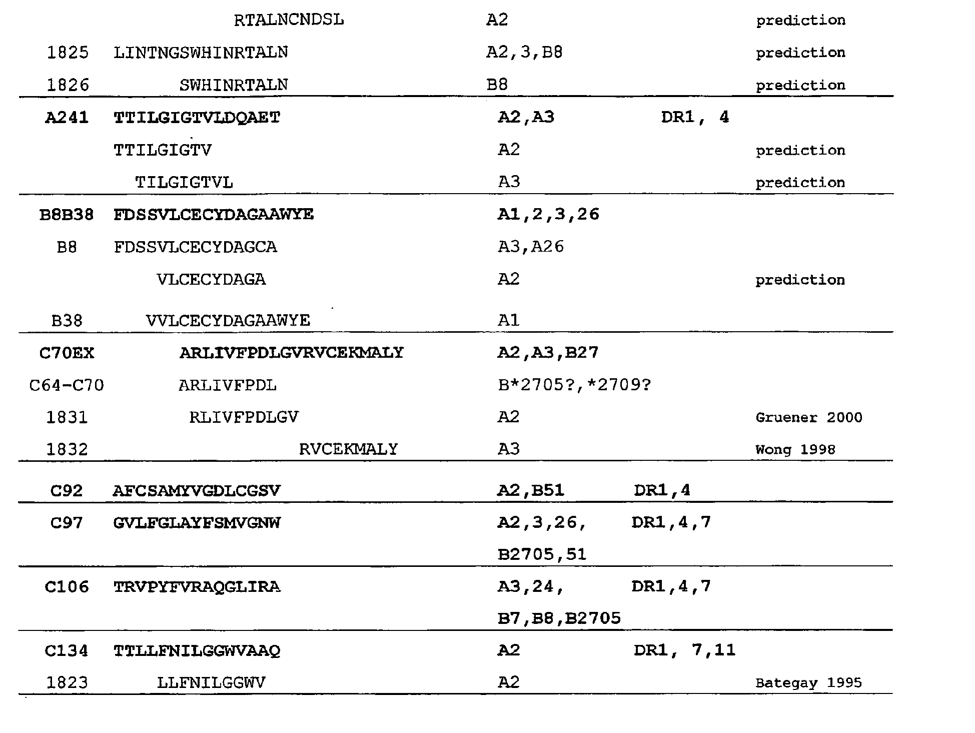

### Example III. HCV epitope hotspot Ipep 1426 contains at least HLA-A*0201 and several promiscuous class II T-cell epitopes

The major objective of this experiment was to compare the immunogenicity of the "hospot" Ipep 1426, which contains at least one HLA-A*0201 epitope (Ipep 1334) and 2 promiscuous class II epitopes (Ipeps 1006 and 1425), to the individual epitopes. To this end peripheral blood mononuclear cells (PBMC) from several healthy HLA-typed blood donors were stimulated in vitro either with 1426 or a mixture of 1334, 1006, 1425. Three rounds of stimulation were performed resulting in oligoclonal T cell lines. Then, responses against all four peptides were assessed by interferon-gamma (IFN-γ) ELIspot analysis.

Peptide 1426, induces T cell responses similarly well as individual epitopes comprised within its sequence. In particular, CD8 positive T cells directed against the HLA-A*0201 restricted epitope 1334 were successfully generated.

**Table 3: peptide induced IFN-γ secretion of oligoclonal T cell lines. Lines were generated from two HLA-typed healthy individuals by 3 rounds of in vitro priming with either peptide 1426 or a mixture of peptides 1006+1425+1334. The reactivity of CD4 and CD8 positive T cells in these lines was assessed by IFN-γ ELIspot ("+++" very strong, "++" strong, "+" significant, "-" no IFN-gamma secretion).**

| **Donor HLA** | **A*0201,** A*03, B7, B60; DR**B1*1501,** -B1*1302 | | A*0206, A*01, B27, B50; DR**B1*0401,** -B1*1402 | |
|---|---|---|---|---|
| **Peptide ID** | **line raised line against 1426** | **raised against 1006+1425+133 4** | **line raised line against 1426** | **raised against 1006+1425+133 4** |
| **1006** | ++ | ++ | ++ | ++ |
| **1425** | +++ | +++ | +++ | ++ |
| **1334** | + | + | - | - |
| | | | | |
| **1006+1425+1334** | ++ | ++ | ++ | ++ |
| **1426** | +++ | +++ | +++ | ++ |
| | | | | |
| **84 (HCV derived negative control)** | - | - | - | - |

### Example IV. Potent HCV-specific type 1 cellular responses are induced by the combined injection of five different HCV-derived peptides, the antimicrobial peptide KLK and the synthetic oligodeoxynucleotide o-d(IC)₁₃.

### Mice HLA-A*0201 transgenic mice (HHD.1)

- Peptides: The peptides p83, p84, p87, p89, p1426 were used for vaccination.
p83: HCV-derived peptide, (KFPGGGQIVGGVYLLPRRGPRL)
p84: HCV-derived peptide, (GYKVLVLNPSVAAT)
p87: HCV-derived peptide, (DLMGYIPAV)
p89: HCV-derived peptide, (CINGVCWTV)
p1426: HCV-derived peptide, (HMWNFISGIQYLAGLSTLPGNPA)
(p1274 used for restimulation as irrelev- ant peptide (YMDGTMSQV; HLA-A*0201 restricted)
All peptides were synthesised by standard solid phase F-moc synthesis, HPLC purified and analyzed by mass spectroscopy for pur- ity.
dose: 20µg per peptide/mouse
- KLK KLKLLLLLKLK-COOH: was synthesised by MPS (Multiple Peptide System, USA)
dose: 10nmol/mouse
- oligo-d(IC)₁₃ (= ODN1a) ODN: 5'ICI CIC ICI CIC ICI CIC ICI CIC IC3' was synthesised by Purimex Nucleic Acids Technology, Göttingen
dose: 0.4nmol/mouse
- Formulation: 10mM Tris/135mM NaCl; pH ∼7

### Experimental setup (5 mice/group):

1. HCV peptides
2. HCV peptides + KLK + o-d(IC)₁₃

On days 0, 14 and 28 HHD.1 mice were injected s.c. into both hind footpads with a total volume of 100µl/mouse (50µl/footpad) containing the above listed compounds. At day 35 (7 days after last vaccination) CD4⁺ as well as CD8⁺ T cells were isolated by magnetic separation (MACS, Miltenyi) from single cell suspensions of spleen cells. T cells were incubated with medium (background control) or were restimulated with irradiated spleen cells from naive HHD.1 mice as APC in the presence of either the different peptides used for vaccination or the irrelevant peptide p1274. After overnight incubation, the IFN-γ production was analyzed using an ELIspot assay.

Figure 1 shows that upon co-injection of the five HCV-derived peptides with KLK/o-d(IC)₁₃ high amounts of IFN-γ produced by CD4⁺ T cells against p84, p87, p89, p1426 were induced. Furthermore, a strong IFN-γ production by CD8⁺ T cells against p87, p89 was detectable.

### Example V: Solubilisation of peptide mixtures (HCV peptides plus poly-L-arginine):

The peptide mixture contains the peptides 83, 84, 87, 89, 1426 and pR as immuniser (see table 4), wherein peptides 83 and 84 are soluble in water and DMSO, peptides 87 and 89 are poorly water soluble, but dissolve easily in DMSO and peptide 1426 is only soluble in DMSO. For the manufacturing of a suspension formulation it is necessary first to dissolve peptides 83 and 84 in an aqueous buffer solution and peptides 87, 89, and 1426 in DMSO. After sterile filtration the solutions can be combined to form the final suspension.

Water/acetonitrile mixtures as well as DMSO and DMF did not work as solvents and resulted in suspensions that could not be sterile filtered due to filter blockage. Surprisingly a 50% acetic acid/water mixture was found to dissolve all components. The obtained peptide solution containing the above mentioned peptides could easily sterile filtered without any filter blocking.

**Table 4 Peptide mixture I**

| ***Peptide ID*** | ***Peptide sequence*** |
|---|---|
| 83 | KFPGGGQIVGGVYLLPRRGPRL |
| 84 | GYKVLVLNPSVAAT |
| 87 | DLMGYIPAV |
| 89 | CINGVCWTV |
| 1426 | HMWNFISGIQYLAGLSTLPGNPA |

The following examples (examples V.1 to V.12, tables 5 to 16) illustrate that peptide mixtures containing at least two peptides with variable length and amino acid composition are suitable to be solubilised according to the present invention. Furthermore the tables show the known affinities of the peptides to HLA class I and class II molecules.

### Example V.1:

**Table 5 Peptide mixture II**

| ***Peptide ID*** | ***Peptide sequence*** | ***HLA class I*** | ***HLA class II*** |
|---|---|---|---|
| 1835 | KFPGGGQIVGGVYLLPRRGPRLGVRATRK | A2, A3, B7 | DR11 |
| 87 | DLMGYIPAV | A2 | |
| 1624 | LEDRDRSELSPLLLSTTEW | B60 | DR7 |
| 1846 | DYPYRLWHYPCTVNFTIFKV | A2, A11, Cw7 | DR1, 4, 7, 11 |
| 84 | GYKVLVLNPSVAAT | | DR1, 4, 7, 11 |
| 89 | CINGVCWTV | A2 | |
| 1799 | AAWYELTPAETTVRLR | B35 | DR1, 4 |
| 1547 | YLVAYQATVCARAQAPPPSWD | A2 | DR1, 4, 7, 11 |
| 1827 | TAYSQQTRGLLG | A24 | DR1, 7, 11 |
| 1426 | HMWNFISGIQYLAGLSTLPGNPA | A2 | DR1, 4, 7, 11 |
| 1798 | IGLGKVLVDILAGYGAGVAGALVAFK | A2, A3, A11 | DR1, 4, 7 |
| 1829 | SMSYTWTGALITP | A2, B7 | DR1, 7, 11 |

### Example V.2:

**Table 6 Peptide mixture III**

| ***Peptide ID*** | ***Peptide sequence*** | ***HLA class I*** | ***HLA class II*** |
|---|---|---|---|
| 1835 | KFPGGGQIVGGVYLLPRRGPRLGVRATRK | A2, A3, B7 | DR11 |
| 1846 | DYPYRLWHYPCTVNFTIFKV | A2, A11, Cw7 | DR1, 4, 7, 11 |
| 1799 | AAWYELTPAETTVRLR | B35 | DR1, 4 |
| 1827 | TAYSQQTRGLLG | A24 | DR1, 7, 11 |
| 1426 | HMWNFISGIQYLAGLSTLPGNPA | A2 | DR1, 4, 7, 11 |
| 1798 | IGLGKVLVDILAGYGAGVAGALVAFK | A2, A3, A11 | DR1, 4, 7 |
| 1829 | SMSYTWTGALITP | A2, B7 | DR1, 7, 11 |

### Example V.3:

**Table 7 Peptide mixture IV**

| ***Peptide ID*** | ***Peptide sequence*** | ***HLA class I*** | ***HLA class II*** |
|---|---|---|---|
| C134 | TTLLFNILGGWVAAQ | A2 | DR1, 7, 11 |
| 1815 | TVNYTIFKI | A11 | |
| 1006 | MWNFISGIQYLAGLSTLPGN | | |

### Example V.4:

**Table 8 Peptide mixture V**

| ***Peptide ID*** | ***Peptide sequence*** | ***HLA class I*** | ***HLA class II*** |
|---|---|---|---|
| 84 | GYKVLVLNPSVAAT | | DR1, 4, 7, 11 |
| 87 | DLMGYIPAV | A2 | |
| 89 | CINGVCWTV | A2 | |
| 1426 | HMWNFISGIQYLAGLSTLPGNPA | A2 | DR1, 4, 7, 11 |

### Example V.5:

**Table 9 Peptide mixture VI**

| ***Peptide ID*** | ***Peptide sequence*** | ***HLA class I*** | ***HLA class II*** |
|---|---|---|---|
| 1051 | YLLPRRGPRL | A2 | |
| 84 | GYKVLVLNPSVAAT | | DR1, 4, 7, 11 |
| 87 | DLMGYIPAV | A2 | |
| 89 | CINGVCWTV | A2 | |
| 1426 | HMWNFISGIQYLAGLSTLPGNPA | A2 | DR1, 4, 7, 11 |

### Example V.6:

**Table 10 Peptide mixture VII**

| ***Peptide ID*** | ***Peptide sequence*** | ***HLA class I*** | ***HLA class II*** |
|---|---|---|---|
| 1006 | MWNFISGIQYLAGLSTLPGN | | |
| 1827EX | GWRLLAPITAYSQQTRGLLGCIV | B8 | |

### Example V.7:

**Table 11 Peptide mixture VIII**

| ***Peptide ID*** | ***Peptide sequence*** | ***HLA class I*** | ***HLA class II*** |
|---|---|---|---|
| 1604 | VVCCSMSYTWTGALITPC | | |
| 83 | KFPGGGQIVGGVYLLPRRGPRL | B8 | |
| A130 | DYPYRLWHYPCTVNF | | |
| B46 | AGAAWYELTPAETTV | | |

### Example V.8:

**Table 12 Peptide mixture IX**

| ***Peptide ID*** | ***Peptide sequence*** | ***HLA class I*** | ***HLA class II*** |
|---|---|---|---|
| B84 | GSIGLGKVLVDILAG | | DR1, 4 |
| B96 | LAGYGAGVAGALVAF | | DR1, 4, 7, 11 |
| 1829 | SMSYTWTGALITP | A2, B7 | DR1, 7, 11 |
| A135 | LWHYPCTVNFTIFKV | | DR7 |
| A130 | DYPYRLWHYPCTVNF | | DR1, 4 |
| 1426 | HMWNFISGIQYLAGLSTLPGNPA | A2 | DR1, 4, 7, 11 |
| 1817 | RMYVGGVEHRL | | DR4 |
| 87EX | DLMGYIPLVGAPL | A2, 24 | |
| 1816 | TINYTIFK | A11 | |

### Example V.9:

**Table 13 Peptide mixture X**

| ***Peptide ID*** | ***Peptide sequence*** | ***HLA class I*** | ***HLA class II*** |
|---|---|---|---|
| 1650 | VDYPYRLWHYPCTVNFTIFKVRMYVG- GVEHRL | Cw7,A2,A24, A11, A3 | DR1, 4, 7, 11 |
| 1836 | DYPYRLWHYPCTVNFTIFKI | Cw7,A2,A24, A11 | DR1, 4, 7, 11 |
| 1846 | DYPYRLWHYPCTVNFTIFKV | Cw7, A2, A24, A1, A11 | DR1,4,7,11 |
| 1651 | VDYPYRLWHYPCTVNYTIFKIRMYVG- GVEHRL | | |
| 1800 | DYPYRLWHYPCTVNYTIFKI | Cw7,A24,A11 | DR7 |

### Example V.10:

**Table 14 Peptide mixture XI**

| ***Peptide ID*** | ***Peptide sequence*** | ***HLA class I*** | ***HLA class II*** |
|---|---|---|---|
| 1835 | KFPGGGQIVGGVYLLPRRGPRLGVRATRK | A2, A3, B7 | DR11 |
| 83 | KFPGGGQIVGGVYLLPRRGPRL | A2 B7 | DR11 |
| 84EX | AYAAQGYKVLVLNPSVAAT | A24 | DR1,4,7,11 |
| 87EX | DLMGYIPLVGAPL | A2, A24 | |
| 89EX | GEVQWSTATQSFLATCINGVCWTV | A2 | DR 4, 7 |
| 1426 | HMWNFISGIQYLAGLSTLPGNPA | A2 | DR1,4,7,11 |

### Example V. 11:

**Table 15 Peptide mixture XII**

| ***Peptide ID*** | ***Peptide sequence*** | ***HLA class I*** | ***HLA class II*** |
|---|---|---|---|
| C114 | TAYSQQTRGLLGCIV | A24, B8 | DR1,4,7,11 |
| 1798 | IGLGKVLVDILAGYGAGVAGALVAFK | A2,24,3,11 | DR1,4,7 |
| 1604 | VVCCSMSYTWTGALITPC | A2 ,A24, B7 | DR1,4,7,11 |
| 1624 | LEDRDRSELSPLLLSTTEW | A1, 2, 3, 26 | DR7 |

### Example V.12:

**Table 16 Peptide mixture XIII**

| ***Peptide ID*** | ***Peptide sequence*** | ***HLA class I*** | ***HLA class II*** |
|---|---|---|---|
| 1547 | YLVAYQATVCARAQAPPPSWD | A2 | DR1,4,7,11 |
| A1A7 | MSTNPKPQRKTKRNTNR | A11,B08,B27 | |
| A122EX | LINTNGSWHINRTALNCNDSL | A2,2,3,B8 | DR1,4,7,11 |
| A241 | TTILGIGTVLDQAET | A2,A3 | DR1, 4 |
| B8B38 | FDSSVLCECYDAGAAWYE | A1,2,3,26 | |
| C70EX | ARLIVFPDLGVRVCEKMALY | A2,A3,B27 | |
| C92 | AFCSAMYVGDLCGSV | A2,B51 | DR1, 4 |
| C97 | GVLFGLAYFSMVGNW | A2,3,26, B2705, 51 | DR1,4,7 |
| C106 | TRVPYFVRAQGLIRA | A3,24,B7,B8,B 2705 | BDR1,4,7 |
| C134 | TTLLFNILGGWVAAQ | A2 | DR1, 7,11 |

### Example VI. Rationale in designing further HCV vaccine variants according to the present invention

**Table 17: preferred combination of "hotspots I"**

| **Peptide ID** | **Peptide sequence** | **HCV antigen** | **HLA alleles** | | | | | | |
|---|---|---|---|---|---|---|---|---|---|
| | | | **HLA class I** | | | **HLA class II** | | | |
| 1835 | KFPGGGQIVGGVYLLPRRGPRLGVRATRK | core | A2, | A3, | B7 | | | | D |
| 87 | DLMGYIPAV | core | A2 | | | R11 | | | |
| 1624 | LEDRDRSELSPLLLSTTEW | E2 | | | B60 | | | DR7 | |
| 1846 | DYPYRLWHYPCTVNFTIFKV | E2 | A2, | A11, | Cw7 | DR1, | 4, | 7, | 11 |
| 84 | GYKVLVLNPSVAAT | NS3 | | | | DR1, | 4, | 7, | 11 |
| 89 | CINGVCWTV | NS3 | A2 | | | | | | |
| 1799 | AAWYELTPAETTVRLR | NS3 | B35 | | | DR1, | 4 | | |
| 1547 | YLVAYQATVCARAQAPPPSWD | NS3 | A2 | | | DR1, | 4, | 7, | 11 |
| 1827 | TAYSQQTRGLLG | NS3 | A24 | | | DR1, 11 | | 7, | |
| 1426 | HMWNFISGIQYLAGLSTLPGNPA | NS4 | A2 | | | DR1, | 4, | 7, | 11 |
| 1798 | IGLGKVLVDILAGYGAGVAGALVAFK | NS4 | A2, | A3, | A11 | DR1, | 4, | 7 | |
| 1829 | SMSYTWTGALITP | NS5 | A2, | B7 | | DR1, 11 | | 7, | |

| | | | | | | | | | |
|---|---|---|---|---|---|---|---|---|---|
| Characteristics of preferred combination of hotspots I: Number of peptides: 12 Potential interactions of peptides via Cysteins: 4 Cys in 3 peptides (1846, 89, 1547); potential formation of homo- and heterodimers and potentially oligomers of peptides. Number HCV antigens covered: 5 (core, E2, NS3, NS4, NS5) HLA class I alleles covered: at least 8 A2 (8 times), A3 (2 times), A11 (2 times), A24 (1 time) B7 (2 times), B35 (1 time), B60 (1 time) Cw7 (1 time) HLA class II covered: multiple by promiscuous peptides DR1 (8 times), DR4 (6 times), DR7 (8 times), DR11 (7 times) | | | | | | | | | |

The preferred combination of hotspots I consists of 12 peptides derived from conserved regions (>80% in genotypes 1, 2, 3) of 5 different HCV antigens. In total at least 8 important HLA class I alleles are covered (1-8 times, each), providing a population coverage of 83-91% in Europe, 89-91% in U.S. Caucasians and 87-89% in Japanese people (estimated on numbers from HLA-prevalence studies in Gjertson and Terasaki, eds., HLA 1998, American Society of Histocompatibility and Immunogenetics, Lenexa, Kansas, USA). Similarly, several important HLA class II alleles are covered, mostly by so called promiscuous peptides (binding to more than one HLA class II allele). Individual particularly prevalent alleles (DR1, 4, 7, 11) are independently covered 6-8 times. Importantly, DR11 is covered 7 times. Presence of this allele has been linked to spontaneous recovery from HCV infection (Thursz et al., 1999 Lancet: 354(9196):2119-24).
The high number of epitopes contained in this combination of hotspots ensures not only broad population coverage, and hence applicability of the vaccine, but also a broad immune response within individuals treated with such a combination. This is a pre-requisite for efficacy, since spontaneous recovery from HCV infection has been linked to a broad immune response typically directed against 10 or more epitopes (Day et al., 2002 J Virol.: 76(24):12584-95).

A T-cell response against multiple epitopes also reduces the risk of development of HCV-epitope-escape variants (HCV viruses, which do not contain the epitope targeted by the immune response anymore), because the more epitopes targeted at the same time, the more difficult for the virus to change them simultaneously. Experiments in chimpanzees have established, that such HCV escape variants are an important mechanism to maintain chronic infection (Weiner et al, 1995 Proc Natl Acad Sci U S A.: 92(7): 2755-9).

Finally, targeting most of the HCV antigens (Core, E2, NS3, 4, 5) instead of for instance only one also provides significant advantages: different antigens may be recognised differently by T-cells, for instance due to differences in antigen processing, or dictated by the particular HLA haplotype of the person). Also, different antigens have different functional importance during the HCV life-cycle.

Combination hotspot I contains also individual epitopes derived from hotspots (Ipep87 derived from Ipep87EX and Ipep89 derived from Ipep89EX). This has the advantage of a deliberate focusing of the desired response on a particular epitope. Using for instance Ipep89 guarantees that an HLA-A2 restricted CTL response is generated, whereas using 89EX may leed to an HLA-A2 restricted CTL response, that may be compromised by a concomitant DR4 or DR7 restricted T-helper response.

Using combinations of hotspots has a number of advantages over using full-length antigens, either in the form of recombinant proteins or as DNA vaccines. As detailed in the preceding paragraph in certain cases using individual epitopes may be advantageous over using the hotspot or the whole antigen. Second, the hotspots disclosed herein have been identified via functional assays using primarily a Th1/Tc1-type reaction (i.e. interferon gamma) as read-out. It is well known that antigens do not only contain such agonist epitopes, but may also encode antagonist ligands. Such antagonists potentially present in whole antigens, but absent in hotspots may impede immunogenicity and efficacy of the vaccine. Next, whole antigens will almost certainly induce a humoral immune response. Such an antibody response has per se little effect on HCV residing within cells, but it can severely compromise the desired T-cell reponse. Hotspots, due to their restricted length, have a much lower probability to induce unwanted antibody responses. Finally, only relatively small parts of HCV antigens are conserved between different genotypes. Thus, whole antigens used in a vaccine may induce responses against non-conserved regions or epitopes, irrelevant for most of the patients. In contrast, the hotspots disclosed herein are derived from regions conserved > 80% among the major HCV genotypes 1, 2 and 3.

**Table 18: preferred combination of "hotspots II"**

| **Peptide ID** | **Peptide sequence** | **HCV antigen** | **HLA alleles** | | | | | |
|---|---|---|---|---|---|---|---|---|
| | | | **HLA class I** | | | **HLA class II** | | |
| 1835 | KFPGGGQIVGGVYLLPRRGPRLGVRATRK | core | A2, | A3, | B7 | | | |
| | | | | | | DR11 | | |
| 1846 | DYPYRLWHYPCTVNFTIFKV | E2 | A2, | A11, | Cw7 | DR1, | 4, | 7, |
| | | | | | | 11 | | |
| 1799 | AAWYELTPAETTVRLR | NS3 | B35 | | | DR1, | 4 | |
| 1827 | TAYSQQTRGLLG | NS3 | A24 | | | DR1, | | 7, |
| | | | | | | 11 | | |
| 1426 | HMWNFISGIQYLAGLSTLPGNPA | NS4 | A2 | | | DR1, | 4, | 7, |
| | | | | | | 11 | | |
| 1798 | IGLGKVLVDILAGYGAGVAGALVAFK | NS4 | A2, | A3, | A11 | DR1, | 4, | 7 |
| 1829 | SMSYTWTGALITP | NS5 | A2, | B7 | | DR1, | | 7, |
| | | | | | | 11 | | |

| | | | | | | | | |
|---|---|---|---|---|---|---|---|---|
| Characteristics of preferred combination of "hotspots II": Number of peptides: 7 Potential interactions of peptides via Cysteins: 1 Cys (1846); potential formation of homodimers of 1846. Number HCV antigens covered: 5 (core, E2, NS3, NS4, NS5) HLA class I alleles covered: at least 7 A2 (5 times), A3 (2 times), A11 (2 times), A24 (1 time) B7 (2 times), B35 (1 time) Cw7 (1 time) HLA class II covered: multiple by promiscuous peptides DR1 (6 times), DR4 (5 times), DR7 (5 times), DR11 (5 times) | | | | | | | | |

The preferred combination of hotspots II consists of 7 peptides derived from conserved regions (>80% in genotypes 1, 2, 3) of 5 different HCV antigens. In total at least 7 important HLA class I alleles are covered (1-5 times), providing a population coverage of 81-90% in Europe, 89-91% in U.S. Caucasians and 85-88% in Japanese people (estimated on numbers from HLA-prevalence studies in Gjertson and Terasaki, eds., HLA 1998, American Society of Histocompatibility and Immunogenetics, Lenexa, Kansas, USA). Similarly, several important HLA class II alleles are covered, mostly by so called promiscuous peptides (binding to more than one HLA class II allele). Individual particularly prevalent alleles (DR1, 4, 7, 11) are independently covered 5-6 times. Importantly, DR11 is covered 5 times. Presence of this allele has been linked to spontaneous recovery from HCV infection (Thursz et al., 1999 Lancet: 354(9196):2119-24).

In contrast to combination I, combination II has a distinct smaller number of peptides (7 versus 12), which has potential advantages in manufacturability, formulation and competitive production of such a product. Importantly, only one peptide with a cystein side-chain remains (1846). Therefore, only homo-dimers of 1846, but no heterodimers or oligomers could be formed in this combination.

At the same time, combination II covers the same 5 HCV antigens and a similar number of both HLA class I and II alleles as combination I. Hence, the arguments discussed in the previous section, hold also true for combination II.

From a formulation development viewpoint, combination II has several advantages over combination I (only 7 instead of 12 components). Most importantly, only one peptide (1846) caries a free cystein side-chain. Thus, only homodimers od 1846, but no hetero- or oligomers might be formed.

### Example VII: T-cell immunogenicity results from the HCV vaccine-102 dose optimisation trial

In the course of a clinical trial a number of peptide/poly-arginine mixtures were applied as vaccine to healthy volunteers. Each group consisted of 12 subjects, positive for HLA-A2. Subjects received 4 vaccinations in monthly intervals (at visits 3 to 6). Blood for immunological analyses was drawn at visits 1 to 6, one month after last vaccination (visit 7) and 3 months after last vaccination (visit 8). In order to monitor the clinical trials, Intercell has set-up state-of-the-art T cell assays to determine immunological endpoints under GLP/GCP compliance: Interferon-gamma ELIspot Assay, T cell Proliferation Assay, HLA-tetramer/FACS assay. These assays allow reliable measurements of epitope-specific T cell responses induced by the therapeutic HCV vaccine. The vaccine-induced T cell immune responses serve as surrogate parameters of efficacy (Keilholz et al., J Immunother. 2002 Mar-Apr;25(2):97-138).

### As primary endpoint T-cell immunogenicity was determined by a T-cell proliferation assay:

The proliferation assay allows detection of peptide-specific T cells in biological samples like human blood. The basis of the assay is that, T cells upon stimulation with a peptide specifically recognised by their T cell receptor, react by secretion of cytokines and subsequent proliferation. Proliferation of cells can be measured by a variety of means; among the most sensitive approaches ranks incorporation of radioactively labeled thymidine into DNA synthesised prior cell division. This reaction can be carried out in a 96-well plate. Each well contains a fixed number of cells, which are cultured in the presence of antigen/peptide for a couple of days. For the last 16-20 hours thymidine labelled with tritium (3H-thymidine) is added. Cells are then harvested onto a filter plate: medium containing free radioactivity is washed away, whereas DNA sticks to the filter. Incorporated radioactivity can be quantified by means of a beta-scintillation counter. The usual output is given as counts-per-minute (cpm).

### As secondary endpoints T-cell immunogenicity was determined by interferon gamma ELIspot

ELIspot allows quantification of peptide-specific, functional (i.e. cytokine-secreting) T cells in biological samples like human blood. The basis of the assay is that, T cells upon stimulation with a peptide specifically recognised by the T cell receptor react by secretion of cytokines like IFN-gamma. This reaction can be carried out in a 96-well plate. The filter-wells of this plate are coated with a Mab specific for IFN-gamma. Consequently, each cell secreting IFN-gamma leaves an IFN-gamma spot, which can be visualised with a subsequent color reaction. Spots can be counted using automated plate readers. Numbers obtained are a measure for the frequency of peptide-specific, IFN-gamma-secreting T cells in the sample.

### As additional secondary endpoint HLA-tetramer/FACS analysis was performed.

HLA class I tetramers, soluble recombinant forms of a complex of HLA molecule and antigenic peptide, bind the antigen-specific T cell receptor used for T cell recognition. By using flow cytometry with fluorescent tetramers, antigen-specific CD8+ T lymphocytes can be reliably enumerated and characterised. The assay uses HLA-A*0201 custom-made iTag™-tetramers produced by Beckman Coulter Immunomics complexed with HCV vaccine class I epitopes.

Subjects were classified as responders if they showed significant T-cell responses at any of visits 4 to 8 and had no response prior treatment.

Maximum Responder Rates (against any peptide, at any of visits 4 to 8, in any of the three T-cell assays of up to 92% versus 25% in a control group of 25 subjects receiving saline were achieved.

### Responder Rates per peptides Ipep89, Ipep84 and Ipep1426:

As example here responses against T-cell epitope hotspot peptides Ipep89 (class I T-cell epitope), Ipep84 and Ipep1426 are shown. These responses were obtained after a cycle of 4 monthly vaccinations. For group B the vaccine contained poly-L-Arginine only. For Group C the vaccine contained a mixture of 5 peptides, including Ipep89, Ipep84 and Ipep1426. For Group G the vaccine contained a mixture of the same 5 peptides as in Group C plus poly-L-Arginine as a fully synthetic T-cell adjuvant. Table 19 shows that in control group B no responders in ELIspot and HLA-tetramer/FACS analysis and 3 responders with in total only 3 positive visits were seen. The latter can be interpreted as the rate of false-positive responders obtained in proliferation assay.
In group C containing only peptides but no poly-L-Arginine, 1 responder against Ipep84 in ELIspot, 5 and 4 responders against Ipep84 and Ipep1426 respectively in proliferation and 4 responders against Ipep89 in HLA-tetramer/FACS analysis were seen. Finally group G (same peptides as in C plus poly-L-arginine) showed responders in all three assays. Importantly, especially consistent (all three peptides) and sustained (see number of total positive visits) ELIspot responses giving an indication for functional T-cells were dependent on poly-L-arginine as synthetic T-cell adjuvant.

**Table 19: Responders per peptide and total number of positive visits in Groups B, C and G**

| | | REV/REIV RESPONDERS | TOTAL POSITIVE VISITS | RPII RESPONDERS | TOTAL POSITIVE VISITS | RFI RESPONDERS | TOTAL POSITIVE VISITS | VACCINE RESPONDERS | TOTAL POSITIVE VISITS |
|---|---|---|---|---|---|---|---|---|---|
| TREATMENT | PEPTIDE | n/N (%) | N | n/N (%) | N | n/N (%) | N | n/N (%) | N |
| | | | | | | | | | |
| B (poly-Arg only) | Ipep89 | 0 | 0 | ---- | -- | 0 | 0 | 0 | 0 |
| | Ipep84 | 0 | 0 | 3 | 3 | ---- | -- | 3 | 3 |
| | Ipep1426 | 0 | 0 | 0 | 0 | ---- | -- | 0 | 0 |
| | | | | | | | | | |
| C (peptide only) | Ipep89 | 0 | 0 | ---- | | 4 | 4 | 4 | 4 |
| | Ipep84 | 1 | 1 | 5 | 14 | ---- | -- | 6 | 15 |
| | Ipep1426 | 0 | 0 | 4 | 9 | ---- | -- | 4 | 9 |
| | | | | | | | | | |
| G (peptide/pnly-Arg) | Ipep89 | 3 | 5 | ---- | | 5 | 5 | 5 | 8 |
| | Ipep84 | 3 | 6 | 6 | 11 | ---- | -- | 8 | 17 |
| | Ipep1426 | 5 | 13 | 5 | 11 | ---- | -- | 7 | 17 |
| | | | | | | | | | |
| KEY: REV/REIV RESPONDERS-ELISPOT PESPONDERS (CLASS I OR CLASS II): | | | | | | | | | |
| n/N (%) - percentage of responders per pepride divided by total number of responders in treatment group | | | | | | | | | |
| RPII RESPOHDERS-T-CELLE PRCLIFEPATION FESPONDERS (CLASS II ONLY): | | | | | | | | | |
| PF 1 RESPONDERS- HLA-TETRAMER/FACS RESPOHDER (CLASS I ONLY); | | | | | | | | | |
| VACCINE PESPONDEP-ANY OF REV/REIV, RPII OR RFI | | | | | | | | | |

| | | | | | | | | | |
|---|---|---|---|---|---|---|---|---|---|
| 1. T-CELL PROLIFERATION RESPONSES ONLY CALCULATED ON IPEP 1426. 2. FACS RESPONSES OHLY CALCULATED ON IPEP 99. | | | | | | | | | |

### Example of an individual course (ELIspot): subject 5 from group G

Table 20 shows the interferon-gamma ELIspot results obtained from serial blood draws of a particular subject from group G (5 peptides+poly-L-arginine). At visit 5, one month after the second vaccination already the two class II epitopes Ipep84, 1426 show responses. After the third vaccination a peak in all responses is reached and also the class I epitope Ipep89 shows a response. Moreover, Ipep1426 shows a sustained response even 3 months after the last vaccination at visit 8.

**Table 20: ELIspot values of subject 5 from group G) for Ipep89, 84, 1426 over time**

| (spots per 1 million PBMC, background subtracted; non-significant values were set to zero)) | | | | | | | |
|---|---|---|---|---|---|---|---|
| | Visit 1 | Visit 3 | Visit 4 | Visit 5 | Visit 6 | Visit 7 | Visit 8 |
| Ipep89 | 0 | 0 | 0 | 0 | 22 | 0 | 0 |
| Ipep84 | 0 | 0 | 0 | 18 | 45 | 0 | 0 |
| Ipep1426 | 0 | 0 | 0 | 22 | 92 | 16 | 16 |
| | | | | | | | |

### References:

Aichinger G et al., 1997. J. Biol. Chem. 272: 29127-29136.
Ausubel FM et al. (editors), 2001. Current Protocols in Molecular Biology, Volumes 1-4, John Wiley and Sons (publishers).
Bellentani S et al., Microbes Infect. 2000 Nov;2(14):1757-63.
Bonifacino JS et al. (editors), 2001. Current Protocols in Cell Biology, Volumes 1-2, John Wiley and Sons (publishers).
Cornberg M et al., Curr Gastroenterol Rep. 2002 Feb;4(1):23-30. Cox AL et al., 1994. Science 264: 716-719
Coligan JE et al. (editors), 2001. Current Protocols in Immunology , Volumes 1-4, John Wiley and sons (publishers).
Farci P et al., Semin Liver Dis.2000;20(1):103-26 Gavin MA et al., 1993. J Immunol. 151: 3971-80
Gorga, JC et al., 1987. J. Biol. Chem. 262: 16087-16094. Heemels, MT et al, 1995. Annu. Rev. Biochem. 64: 463-491
Kern F et al, 2000. Eur J Immun 30: 1676-1682
Kern F et al, 1999. J Virol 73(10): 8179-8184
Klein, J, 1986. Natural History of the MHC, John Wiley and Sons (publishers)
Kronenberg M et al., 1999. Immunol Today 20: 515-21.
Kwok WW et al., 2001. Trends Immunol 22(11): 583-588.
Lalvani A et al., 1997. J. Exp. Med. 186 (6): 859-865.
Liang TJ et al., Ann Intern Med. 2000 Feb 15;132(4):296-305.
Maecker HT et al, 2001. J Immunol Methods 255: 27-40
Nijman HW et al, 1993. Eur. J. Immunol., 6, 1215-9
Novak N et al., 2001. J. Immunol. 166: 6665-6670.
Parker, KC et al., 1994. J. Immunol. 152: 163.
Rammensee, HG et al., 1999. Immunogenetics 50: 213-219
Stern LJ et al., 1994. Structure 2: 245-251
Sturniolo T et al., 1999. Nature Biotechnology 17: 555-562.
Tobery WT et al., 2001. J Immunol Methods 254: 59-66.
Valli A et al., 1993. J. Clin. Invest. 91: 616-628
Van den Eynde BJ, et al., 1997. Curr Opin Immunol. 5: 684-93
Villadangos JA et al., 2000. Immunity 12: 233-239
Ward S et al., Clin Exp Immunol. 2002 May;128(2):195-203.
Wilson DB et al., 1999. J. Immunol. 163: 6424-6434.

## Claims

1. Hepatitis C virus (HCV) vaccine comprising at least the following three peptides:
a) AYAAQGYKVLVLNPSVAAT or a part thereof containing at least one epitope; and
b) GEVQVVSTATQSFLATCINGVCWTV or a part thereof containing at least one epitope; and
c) HMWNFISGIQYLAGLSTLPGNPA or a part thereof containing at least one epitope.

2. HCV vaccine according to claim 1 **characterised in that** it comprises the following peptides:
GYKVLVLNPSVAAT,
DLMGYIPAV,
CINGVCWTV and
HMWNFISGIQYLAGLSTLPGNPA

3. HCV vaccine according to claim 1 or 2, **characterised in that** it comprises the peptide KFPGGGQIVGGVYLLPRRGPRLGVRATRK.

4. HCV vaccine according to claim 1, **characterised in that** it comprises the peptide DLMGYIPAV.

5. HCV vaccine according to claim 1, **characterised in that** it comprises the peptide CINGVCWTV as an epitope containing part of GEVQVVSTATQSFLATCINGVCWTV.

6. HCV vaccine according to claim 1, **characterised in that** it comprises the peptide HMWNFISGIQYLAGLSTLPGNPA.

7. HCV vaccine according to claim 1, **characterised in that** it comprises the peptide GYKVLVLNPSVAAT as an epitope containing part of AYAAQGYKVLVLNPSVAAT.

8. HCV vaccine according to any one of claims 1 to 7, **characterised in that** it further contains an immunostimulatory peptide comprising a sequence R₁-XZXZ_{N}XZX-R₂, whereby N is a whole number between 3 and 7, preferably 5, X is a positively charged natural and/or non-natural amino acid residue, Z is an amino acid residue selected from the group consisting of L, V, I, F and/or W, and R₁ and R₂ are selected independently one from the other from the group consisting of -H, -NH₂, -COCH₃, -COH, a peptide with up to 20 amino acid residues or a peptide reactive group or a peptide linker with or without a peptide; X-R₂ may be an amide, ester or thioester of the C-terminal amino acid residue of the peptide ("Peptide A").

9. HCV vaccine according to any one of claims 1 to 8, **characterised in that** it further contains an immunostimulatory oligodeoxynucleic acid molecule (ODN) having the structure according to the formula (I) 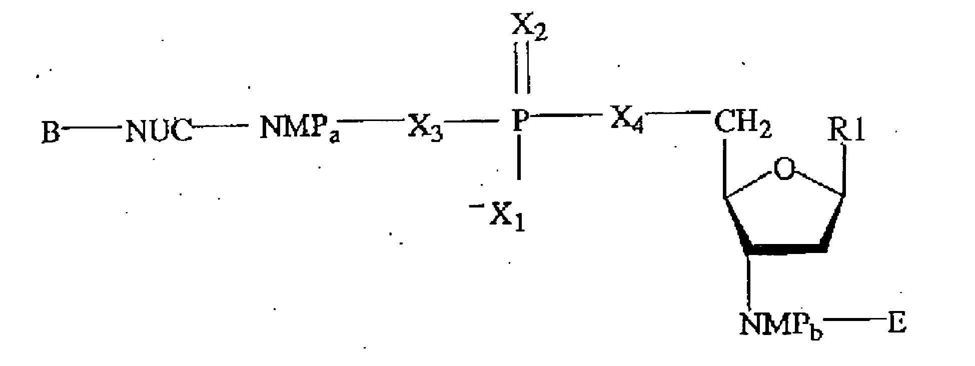 wherein
R1 is selected from hypoxanthine and uracile,
any X is O or S,
any NMP is a 2' deoxynucleoside monophosphate or monothiophosphate, selected from the group consisting of deoxyadenosine-, deoxyguanosine-, deoxyinosine-, deoxycytosine-, deoxyuridine-, deoxythymidine-, 2-methyl-deoxyinosine-, 5-methyl-deoxycytosine-, deoxypseudouridine-, deoxyribosepurine-, 2-amino-deoxyribosepurine-, 6-S-deoxyguanine-, 2-dimethyl-deoxyguanosine- or N-isopentenyl-deoxyadenosine-monophosphate or -monothiophosphate,
NUC is a 2' deoxynucleoside, selected from the group consisting of deoxyadenosine-, deoxyguanosine-, deoxyinosine-, deoxycytosine-, deoxyinosine-, deoxythymidine-, 2-methyl-deoxyuridine-, 5-methyl-deoxycytosine-, deoxypseudouridine-, deoxyribosepurine-, 2-amino-deoxyribosepurine-, 6-S-deoxyguanine-, 2-dimethyl-deoxyguanosine- or N-isopentenyl-deoxyadenosine,
a and b are integers from 0 to 100 with the proviso that a + b is between 4 and 150, and
B and E are common groups for 5' or 3' ends of nucleic acid molecules ("I-/U-ODN").

10. HCV vaccine according to any one of claims 1 to 9, **characterised in that** it further contains an Al(OH)₃ adjuvant.

11. HCV vaccine according to any one of claims 1 to 10, **characterised in that** it further contains an immunostimulatory polycationic peptide.

12. HCV vaccine according to any one of claims 8 to 11, **characterised in that** said Peptide A is KLKL₅KLK.

13. HCV vaccine according to any one of claims 9 to 12, **characterised in that** said I-/U-ODN is oligo d(IC)₁₃.

14. HCV vaccine according to any one of claims 1 to 13, **characterised in that** it further contains an immunostimulatory oligodeoxynucleotide containing a CpG-motif.

15. HCV vaccine according to any one of claims 1 to 14, **characterised in that** it is lyophilised in a form which is recontitutable within 15 min. at 37°C.

16. HCV vaccine according to any one of claims 1 to 15, **characterised in that** it contains between 20 µg and 10 mg of each peptide.

17. HCV vaccine according to any one of claims 1 to 16, **characterised in that** it is lyophilised and contains traces of acetic acid.

18. Use of a vaccine according to any one of claims 1 to 17 for the preparation of a medicament for the prevention and treatment of an infection with HCV.

19. Method for the preparation of a vaccine according to any one of claims 1 to 17, **characterised by** the following steps:
- chemically synthesising the at least three peptides as defined in claims 1 to 17,
- solubilising these peptides by an aqueous solution containing at least one organic acid selected from the group consisting of formic acid, acetic acid, propionic acid, butyric acid and halogenated or hydroxylated forms thereof,
- mixing the solubilised peptides and
- optionally lyophilising the mixed peptides.

## Patentansprüche

1. Hepatitis C-Virus (HCV)-Vakzine umfassend mindestens die folgenden drei Peptide:
a) AYAAQGYKVLVLNPSVAAT oder einen Teil davon, welcher mindestens ein Epitop enthält; und
b) GEVQVVSTATQSFLATCINGVCWTV oder einen Teil davon, welcher mindestens ein Epitop enthält; und
c) HMWNFISGIQYLAGLSTLPGNPA oder einen Teil davon, welcher mindestens ein Epitop enthält.

2. HCV-Vakzine gemäß Anspruch 1, **dadurch gekennzeichnet, dass** sie die folgenden Peptide umfasst:
GYKVLVLNPSVAAT,
DLMGYIPAV,
CINGVCWTV und
HMWNFISGIQYLAGLSTLPGNPA.

3. HCV-Vakzine gemäß Anspruch 1 oder 2, **dadurch gekennzeichnet, dass** sie das Peptid KFPGGGQIVGGVYLLPRRGPRLGVRATRK umfasst.

4. HCV-Vakzine gemäß Anspruch 1, **dadurch gekennzeichnet, dass** sie das Peptid DLMGYIPAV umfasst.

5. HCV-Vakzine gemäß Anspruch 1, **dadurch gekennzeichnet, dass** sie das Peptid CINGVCWTV als ein Epitop umfasst, welches einen Teil von GEVQVVSTATQSFLATCINGVCWTV enthält.

6. HCV-Vakzine gemäß Anspruch 1, **dadurch gekennzeichnet, dass** sie das Peptid HMWNFISGIQYLAGLSTLPGNPA umfasst.

7. HCV-Vakzine gemäß Anspruch 1, **dadurch gekennzeichnet, dass** sie das Peptid GYKVLVLNPSVAAT als ein Epitop umfasst, welches einen Teil von AYAAQGYKVLVLNPSVAAT enthält.

8. HCV-Vakzine gemäß einem der Ansprüche 1 bis 7, **dadurch gekennzeichnet, dass** sie des Weiteren ein immunstimulatorisches Peptid enthält, welches eine Sequenz R₁-XZXZ_{N}XZX-R₂ umfasst, wobei N eine ganze Zahl zwischen 3 und 7, bevorzugt 5, ist, X ein positiv geladener natürlicher und/oder nicht natürlicher Aminosäurerest ist, Z ein Aminosäurerest ausgewählt aus der Gruppe bestehend aus L, V, I, F und/oder W ist, und R₁ und R₂ voneinander unabhängig ausgewählt sind aus der Gruppe bestehend aus -H, -NH₂, -COCH₃, -COH, einem Peptid mit bis zu 20 Aminosäureresten oder einer peptidreaktiven Gruppe oder einem Peptidlinker mit oder ohne Peptid; X-R₂ ein Amid, Ester oder Thioester des C-terminalen Aminosäurerests des Peptids ("Peptid A") sein kann.

9. HCV-Vakzine gemäß einem der Ansprüche 1 bis 8, **dadurch gekennzeichnet, dass** sie des Weiteren ein immunstimulatorisches Oligodesoxynukleinsäuremolekül (ODN) mit der Struktur gemäß der Formel (I) enthält, 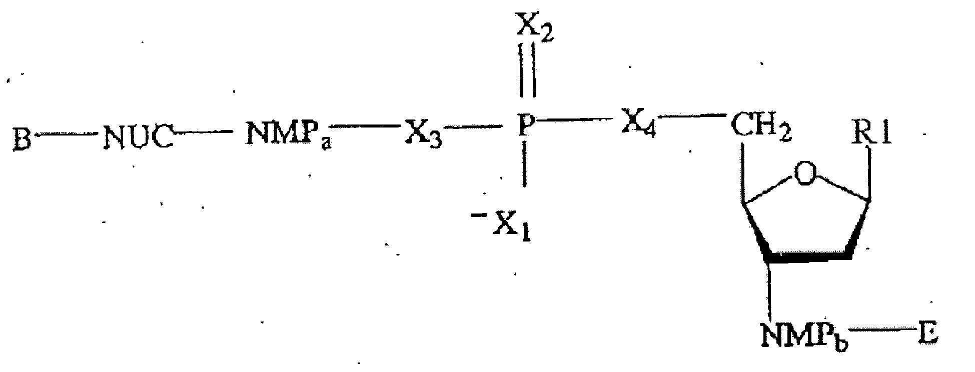 wobei
R1 ausgewählt ist aus Hypoxanthin und Uracil,
jedes X ein O oder S ist,
jedes NMP ein 2'-Desoxynukleosid-Monophosphat oder -Monothiophosphat ist, ausgewählt aus der Gruppe bestehend aus Desoxyadenosin-, Desoxyguanosin-, Desoxyinosin-, Desoxycytosin-, Desoxyuridin-, Desoxythymidin-, 2-Methyl-Desoxyinosin-, 5-Methyl-Desoxycytosin-, Desoxypseudouridin-, Desoxyribosepurin-, 2-Amino-Desoxyribosepurin-, 6-S-Desoxyguanin-, 2-Dimethyl-Desoxyguanosin- oder N-Isopentenyl-Desoxyadenosin-Monophosphat oder -Monothiophosphat,
NUC ein 2' Desoxynukleosid ist, ausgewählt aus der Gruppe bestehend aus Desoxyadenosin-, Desoxyguanosin-, Desoxyinosin-, Desoxycytosin-, Desoxyinosin-, Desoxythymidin-, 2-Methyl-Desoxyuridin-, 5-Methyl-Desoxycytosin-, Desoxypseudouridin-, Desoxyribosepurin-, 2-Amino-Desoxyribosepurin-, 6-S-Desoxyguanin-, 2-Dimethyl-Desoxyguanosin- oder N-Isopentenyl-Desoxyadenosin,
a und b ganze Zahlen zwischen 0 und 100 sind, mit der Maßgabe, dass a + b zwischen 4 und 150 liegt, und
B und E übliche Gruppen für die 5'- oder 3'-Enden von Nukleinsäuremolekülen sind ("I-/U-ODN").

10. HCV-Vakzine gemäß einem der Ansprüche 1 bis 9, **dadurch gekennzeichnet, dass** sie des Weiteren ein Al (OH)₃-Adjuvans enthält.

11. HCV-Vakzine gemäß einem der Ansprüche 1 bis 10, **dadurch gekennzeichnet, dass** sie des Weiteren ein immunstimulatorisches polykationisches Peptid enthält.

12. HCV-Vakzine gemäß einem der Ansprüche 8 bis 11, **dadurch gekennzeichnet, dass** es sich bei dem Peptid A um KLKL₅KLK handelt.

13. HCV-Vakzine gemäß einem der Ansprüche 9 bis 12, **dadurch gekennzeichnet, dass** es sich bei dem I-/U-ODN um Oligo-d (IC)₁₃ handelt.

14. HCV-Vakzine gemäß einem der Ansprüche 1 bis 13, **dadurch gekennzeichnet, dass** sie des Weiteren ein ein CpG-Motiv enthaltendes immunstimulatorisches Oligodesoxynukleotid enthält.

15. HCV-Vakzine gemäß einem der Ansprüche 1 bis 14, **dadurch gekennzeichnet, dass** sie in einer Form lyophilisiert ist, die innerhalb von 15 min bei 37°C rekonstituiert werden kann.

16. HCV-Vakzine gemäß einem der Ansprüche 1 bis 15, **dadurch gekennzeichnet, dass** sie zwischen 20 µg und 10 mg eines jeden Peptids enthält.

17. HCV-Vakzine gemäß einem der Ansprüche 1 bis 16, **dadurch gekennzeichnet, dass** sie lyophilisiert ist und Spuren von Essigsäure enthält.

18. Verwendung einer Vakzine gemäß einem der Ansprüche 1 bis 17 zur Herstellung eines Medikaments zur Prävention und Behandlung einer HCV-Infektion.

19. Verfahren zur Herstellung einer Vakzine gemäß einem der Ansprüche 1 bis 17, **gekennzeichnet durch** die folgenden Schritte:
- chemische Synthese der mindestens drei Peptide, wie in den Ansprüchen 1 bis 17 definiert,
- Solubilisieren dieser Peptide mittels einer wässrigen Lösung enthaltend mindestens eine organische Säure ausgewählt aus der Gruppe bestehend aus Ameisensäure, Essigsäure, Propionsäure, Buttersäure sowie halogenierten und hydroxylierten Formen davon,
- Vermischen der solubilisierten Peptide und
- optionale Lyophilisierung der vermischten Peptide.

## Revendications

1. Vaccin contre le virus de l'hépatite C (HCV [*Hepatitis C virus*]) comprenant au moins les trois peptides suivants :
a) AYAAQGYKVLVLNPSVAAT ou une partie de celui-ci contenant au moins un épitope ; et
b) GEVQVVSTATQSFLATCINGVCWTV ou une partie de celui-ci contenant au moins un épitope ; et
c) HMWNFISGIQYLAGLSTLPGNPA ou une partie de celui-ci contenant au moins un épitope.

2. Vaccin contre le HCV selon la revendication 1, **caractérisé en ce qu'**il comprend les peptides suivants :
GYKVLVLNPSVAAT,
DLMGYIPAV,
CINGVCWTV et
HMWNFISGIQYLAGLSTLPGNPA.

3. Vaccin contre le HCV selon la revendication 1 ou 2, **caractérisé en ce qu'**il comprend le peptide KFPGGGQIVGGVYLLPRRGPRLGVRATRK.

4. Vaccin contre le HCV selon la revendication 1, **caractérisé en ce qu'**il comprend le peptide DLMGYIPAV.

5. Vaccin contre le HCV selon la revendication 1, **caractérisé en ce qu'**il comprend le peptide CINGVCWTV en tant qu'épitope contenant une partie de GEVQVVSTATQSFLATCINGVCWTV.

6. Vaccin contre le HCV selon la revendication 1, **caractérisé en ce qu'**il comprend le peptide HMWNFISGIQYLAGLSTLPGNPA.

7. Vaccin contre le HCV selon la revendication 1, **caractérisé en ce qu'**il comprend le peptide GYKVLVLNPSVAAT en tant qu'épitope contenant une partie de AYAAQGYKVLVLNPSVAAT.

8. Vaccin contre le HCV selon l'une quelconque des revendications 1 à 7, **caractérisé en ce qu'**il contient en outre un peptide immunostimulant comprenant une séquence R₁-XZXZ_{N}XZX-R₂, dans laquelle N est un nombre entier compris entre 3 et 7, de préférence 5, X est un résidu d'acide aminé naturel et/ou non naturel chargé positivement, Z est un résidu d'acide aminé choisi dans le groupe constitué de L, V, I, F et/ou W, et R₁ et R₂ sont choisis, indépendamment l'un de l'autre, dans le groupe constitué de -H, -NH₂, -COCH₃, -COH, un peptide ayant jusqu'à 20 résidus d'acide aminé ou un groupe réactif peptidique ou un lieur de peptide avec ou sans un peptide ; X-R₂ peut être un amide, un ester ou un thioester du résidu d'acide aminé C-terminal du peptide ("Peptide A").

9. Vaccin contre le HCV selon l'une quelconque des revendications 1 à 8, **caractérisé en ce qu'**il contient en outre une molécule d'acide oligodésoxynucléique immunostimulant (ODN) ayant la structure selon la formule (I) 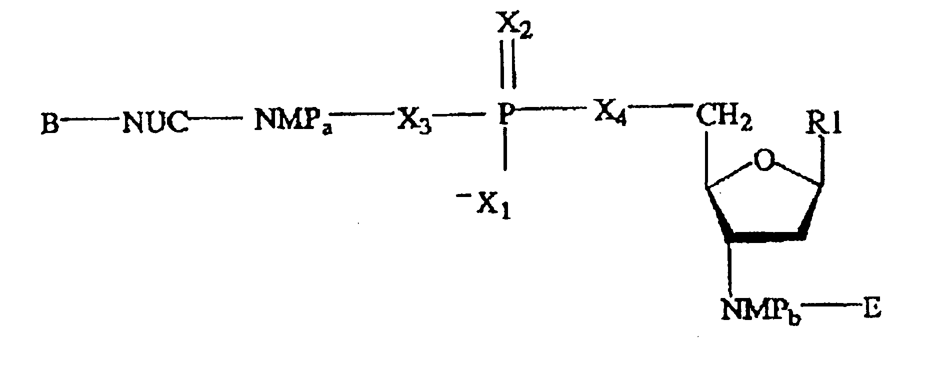 dans laquelle
R1 est choisi parmi l'hypoxanthine et l'uracile,
tout X est O ou S,
tout NMP est un 2' désoxynucléoside monophosphate ou monothiophosphate, choisi dans le groupe constitué de désoxyadénosine-, désoxyguanosine-, désoxyinosine-, désoxycytosine-, désoxyuridine-, désoxythymidine-, 2-méthyl-désoxyinosine-, 5-méthyl-désoxycytosine-, désoxypseudouridine-, désoxyribosépurine-, 2-amino-désoxyribosépurine-, 6-S-désoxyguanine-, 2-diméthyl-désoxyguanosine- ou N-isopentényl-désoxyadénosine-monophosphate ou -monothiophosphate,
NUC est un 2' désoxynucléoside, choisi dans le groupe constitué de désoxyadénosine, désoxyguanosine, désoxyinosine, désoxycytosine, désoxyinosine, désoxythymidine, 2-méthyl-désoxyuridine, 5-méthyl-désoxycytosine, désoxypseudouridine, désoxyribosépurine, 2-amino-désoxyribosépurine, 6-S-désoxyguanine, 2-diméthyl-désoxyguanosine ou N-isopentényl-désoxyadénosine,
a et b sont des nombres entiers de 0 à 100 à condition que a + b soit compris entre 4 et 150, et
B et E sont des groupes communs pour les extrémités 5' ou 3' de molécules d'acide nucléique ("I-/U-ODN").

10. Vaccin contre le HCV selon l'une quelconque des revendications 1 à 9, **caractérisé en ce qu'**il contient en outre un adjuvant Al(OH)₃.

11. Vaccin contre le HCV selon l'une quelconque des revendications 1 à 10, **caractérisé en ce qu'**il contient en outre un peptide polycationique immunostimulant.

12. Vaccin contre le HCV selon l'une quelconque des revendications 8 à 11, **caractérisé en ce que** ledit Peptide A est KLKL₅KLK.

13. Vaccin contre le HCV selon l'une quelconque des revendications 9 à 12, **caractérisé en ce que** ledit I-/U-ODN est un oligo d(IC)₁₃.

14. Vaccin contre le HCV selon l'une quelconque des revendications 1 à 13, **caractérisé en ce qu'**il contient en outre un oligodésoxynucléotide immunostimulant contenant un motif CPG.

15. Vaccin contre le HCV selon l'une quelconque des revendications 1 à 14, **caractérisé en ce qu'**il est lyophilisé sous une forme qui est susceptible d'être reconstituée en 15 min. à 37°C.

16. Vaccin contre le HCV selon l'une quelconque des revendications 1 à 15, **caractérisé en ce qu'**il contient entre 20 µg et 10 mg de chaque peptide.

17. Vaccin contre le HCV selon l'une quelconque des revendications 1 à 16, **caractérisé en ce qu'**il est lyophilisé et qu'il contient des traces d'acide acétique.

18. Utilisation d'un vaccin selon l'une quelconque des revendications 1 à 17 pour la préparation d'un médicament destiné à la prévention et au traitement d'une infection par HCV.

19. Procédé pour la préparation d'un vaccin selon l'une quelconque des revendications 1 à 17, **caractérisé par** les étapes suivantes :
- la synthèse par voie chimique des au moins trois peptides tels que définis dans les revendications 1 à 17,
- la solubilisation de ces peptides à l'aide d'une solution aqueuse contenant au moins un acide organique choisi dans le groupe constitué de l'acide formique, de l'acide acétique, de l'acide propionique, de l'acide butyrique et de leurs formes halogénées ou hydroxylées,
- le mélange des peptides solubilisés et
- éventuellement la lyophilisation des peptides mélangés.
